# FASCICULE DE BREVET EUROPEEN

(11) **EP 2 304 040 B1**
(45) Date de publication et mention de la délivrance du brevet: **06.09.2017**
(21) Numéro de dépôt: 09772760.6
(22) Date de dépôt: 06.07.2009
(51) Int. Cl.: C12P 5/02, C12N 15/63, C12N 9/88, C12N 15/60, C12N 1/20, C12N 1/21

(54) **PRODUCTION D'ALCENES PAR DECARBOXYLATION ENZYMATIQUE D'ACIDES 3-HYDROXY-ALCANOÏQUES**
HERSTELLUNG VON ALKENEN DURCH ENZYMATISCHE DECARBOXYLIERUNG VON 3-HYDROXYALKANSÄUREN
PRODUCTION OF ALKENES BY ENZYMATIC DECARBOXYLATION OF 3-HYDROXYALKANOIC ACIDS

(30) Priorité: 04.07.2008 FR 0854550; 08.07.2008 US 78824 P
(43) Date de publication de la demande: 06.04.2011
(73) Titulaire: Scientist of Fortune S.A., 1628 Luxembourg (LU)
(72) Inventeur: Marlière, Philippe, 7500 Tournai (BE)
(74) Mandataire: Vossius & Partner Patentanwälte Rechtsanwälte mbB
(86) Numéro de dépôt international: PCT/FR2009/051332
(87) Numéro de publication internationale: WO 2010/001078

(56) Documents cités:
- EP-A2- 0 178 153
- EP-A2- 0 205 303
- WO-A2-02/099095
- STINSON R A ET AL: "Beta-alanine as an ethylene precursor. Investigations towards preparation, and properties, of a soluble enzyme system from a subcellular particulate fraction of bean cotyledons" PLANT PHYSIOLOGY, vol. 44, no. 9, 1969, pages 1217-1226, XP002526026 ROCKVILLE, Maryland (US) ISSN: 0032-0889
- THOMPSON J E AND SPENCER M: "Preparation and properties of an enzyme system for ethylene production" NATURE, vol. 210, no. 5036, 7 mai 1966 (1966-05-07), pages 595-597, XP002526027 LONDON (U.K.)
- SHIMOKAWA K ET AL: "The role of beta-hydroxy propionate in ethylene biosynthesis, part 2. Ethylene formation from propionate-2-carbon-14 in banana pulp slices and homogenates" AGRICULTURAL AND BIOLOGICAL CHEMISTRY, vol. 34, no. 11, 1970, pages 1640-1645, XP002526028 ISSN: 0002-1369
- DATABASE UniProt [Online] 5 juillet 2004 (2004-07-05), XP002579929 Database accession no. Q6KZB1_PICTO & FÜTTERER O ET AL: "Genome sequence of Picrophilus torridus and its implications for life around pH 0." PROCEEDINGS OF THE NATIONAL ACADEMY OF SCIENCES OF THE UNITED STATES OF AMERICA, vol. 101, no. 24, 15 juin 2004 (2004-06-15), pages 9091-9096, ISSN: 0027-8424
- JABALQUINTO A M ET AL: "Substrate binding order in mevalonate 5-diphosphate decarboxylase from chicken liver" BIOCHIMICA ET BIOPHYSICA ACTA - PROTEIN STRUCTURE AND MOLECULAR ENZYMOLOGY, vol. 996, no. 3, 6 juillet 1989 (1989-07-06), pages 257-259, XP023579897 ELSEVIER SCIENCE BV, AMSTERDAM, NL ISSN: 0167-4838 [extrait le 1989-07-06] cité dans la demande
- MEIER I K ET AL: "Olefin synthesis by vanadium(V)-induced oxidative decarboxylation- deoxygenation of 3-hydroxy carboxylic acids" JOURNAL OF ORGANIC CHEMISTRY, vol. 55, 1 janvier 1990 (1990-01-01), pages 5619-5624, XP002249270 AMERICAN CHEMICAL SOCIETY, EASTON.; US ISSN: 0022-3263
- TOTH M J ET AL: "Molecular cloning and expression of the cDNAs encoding human and yeast mevalonate pyrophosphate decarboxylase." JOURNAL OF BIOLOGICAL CHEMISTRY, vol. 271, no. 14, 5 avril 1996 (1996-04-05), pages 7895-7898, XP000612170 AMERICAN SOCIETY OF BIOLOCHEMICAL BIOLOGISTS, BIRMINGHAM, US ISSN: 0021-9258
- DATABASE BIOSIS [Online] BIOSCIENCES INFORMATION SERVICE, PHILADELPHIA, PA, US; 1977, WATANABE T ET AL: "AFFINITY CHROMATOGRAPHY OF AN ETHYLENE SYNTHESIZING ENZYME FROM RED ALGA PORPHYRA-TENERA ON AN IMMOBILIZED INHIBITOR OF ETHYLENE EVOLUTION" XP002579930 Database accession no. PREV197764059311 & PLANT AND CELL PHYSIOLOGY, vol. 18, no. 2, 1977, pages 387-392, ISSN: 0032-0781
- DATABASE BIOSIS [Online] BIOSCIENCES INFORMATION SERVICE, PHILADELPHIA, PA, US; avril 2000 (2000-04), REN QUN ET AL: "Properties of engineered poly-3-hydroxyalkanoates produced in recombinant Escherichia coli strains" XP002579931 Database accession no. PREV200000216573 & APPLIED AND ENVIRONMENTAL MICROBIOLOGY, vol. 66, no. 4, avril 2000 (2000-04), pages 1311-1320, ISSN: 0099-2240
- LADYGINA N ET AL: "A review on microbial synthesis of hydrocarbons", PROCESS BIOCHEMISTRY, ELSEVIER, NL, vol. 41, no. 5, 1 May 2006 (2006-05-01), pages 1001-1014, XP027984109, ISSN: 1359-5113 [retrieved on 2006-05-01]
- TOKIWA ET AL: "Biotechnological production of (R)-3-hydroxybutyric acid monomer", JOURNAL OF BIOTECHNOLOGY, ELSEVIER SCIENCE PUBLISHERS, AMSTERDAM, NL, vol. 132, no. 3, 26 October 2007 (2007-10-26), pages 264-272, XP022315138, ISSN: 0168-1656, DOI: 10.1016/J.JBIOTEC.2007.03.015
- ALEXANDER STEINBÜCHEL ET AL: "Synthesis and production of poly(3-hydroxyvaleric acid) homopolyester by", APPLIED MICROBIOLOGY AND BIOTECHNOLOGY, SPRINGER, BERLIN, DE, vol. 39, no. 4 - 5, 1 July 1993 (1993-07-01), pages 443-449, XP035173166, ISSN: 1432-0614, DOI: 10.1007/BF00205030
- MADISON L L ET AL: "METABOLIC ENGINEERING OF POLY(3-HYDROXYALKANOATES): FROM DNA TO PLASTIC", MICROBIOLOGY AND MOLECULAR BIOLOGY REVIEWS, AMERICAN SOCIETY FOR MICROBIOLOGY, US, vol. 63, no. 1, 1 March 1999 (1999-03-01), pages 21-53, XP000869647, ISSN: 1092-2172
- K Zhao ET AL: "Production of ?-(-)-3-hydroxyalkanoic acid by recombinant Escherichia coli", FEMS Microbiology Letters, vol. 218, no. 1, 21 January 2003 (2003-01-21), pages 59-64, XP055208879, ISSN: 0378-1097, DOI: 10.1016/S0378-1097(02)01108-4
- TSENG HSIEN-CHUNG ET AL: "Metabolic Engineering of Escherichia coli for Enhanced Production of (R)- and (S)-3-Hydroxybutyrate", APPLIED AND ENVIRONMENTAL MICROBIOLOGY, AMERICAN SOCIETY FOR MICROBIOLOGY, US, vol. 75, no. 10, 1 May 2009 (2009-05-01), pages 3137-3145, XP002583391, ISSN: 0099-2240, DOI: 10.1128/AEM.02667-08 [retrieved on 2009-03-20]
- SNELL K D ET AL: "Polyhydroxyalkanoate polymers and their production in transgenic plants", METABOLIC ENGINEERING, ACADEMIC PRESS, US, vol. 4, no. 1, 1 January 2002 (2002-01-01) , pages 29-40, XP002395422, ISSN: 1096-7176, DOI: 10.1006/MBEN.2001.0214
- Experimental report provided by the Applicant
- A Hoppensack ET AL: "Analysis of 4-phosphopantetheinylation of polyhydroxybutyrate synthase from Ralstonia eutropha: generation of beta-alanine auxotrophic Tn5 mutants and cloning of the panD gene region", Journal of bacteriology, 1 March 1999 (1999-03-01), pages 1429-1435, XP055208883, UNITED STATES Retrieved from the Internet: URL:http://www.ncbi.nlm.nih.gov/pubmed/100 49372
- NAWRATH C ET AL: "TARGETING OF THE POLYHYDROXYBUTYRATE BIOSYNTHETIC PATHWAY TO THE PLASTIDS OF ARABIDOPSIS THALIANA RESULTS IN HIGH LEVELS OF POLYMER ACCUMULATION", PROCEEDINGS OF THE NATIONAL ACADEMY OF SCIENCES, NATIONAL ACADEMY OF SCIENCES, US, vol. 91, 1 December 1994 (1994-12-01), pages 12760-12764, XP002045228, ISSN: 0027-8424, DOI: 10.1073/PNAS.91.26.12760
- HOUMIEL KATHRYN L ET AL: "Poly(beta-hydroxybutyrate) production in oilseed leukoplasts of Brassica napus", PLANTA, SPRINGER VERLAG, DE, vol. 209, no. 4, 1 October 1999 (1999-10-01), pages 547-550, XP002195214, ISSN: 0032-0935, DOI: 10.1007/S004250050760
- GUO-QIANG CHEN ET AL: "Microbial production and applications of chiral hydroxyalkanoates", APPLIED MICROBIOLOGY AND BIOTECHNOLOGY, SPRINGER, BERLIN, DE, vol. 67, no. 5, 1 June 2005 (2005-06-01), pages 592-599, XP019331858, ISSN: 1432-0614, DOI: 10.1007/S00253-005-1917-2
- Mei-Qing Yuan ET AL: "Microbial production of medium-chain-length 3-hydroxyalkanoic acids by recombinant Pseudomonas putida KT2442 harboring genes fadL, fadD and phaZ", FEMS Microbiology Letters, vol. 283, no. 2, 1 June 2008 (2008-06-01), pages 167-175, XP055208884, ISSN: 0378-1097, DOI: 10.1111/j.1574-6968.2008.01164.x

## Description

### Introduction

La présente invention décrit un procédé de génération d'alcènes par voie biologique. Elle concerne plus particulièrement un procédé de production d'alcènes terminaux (notamment de propylène, d'éthylène, de 1-butylène, d'iso-butylène ou d'iso-amylène) à partir de molécules de type 3-hydroxy-alcanoate.

### Arrière Plan de l'invention

De la pétrochimie sont actuellement dérivés un grand nombre de composés chimiques. Les alcènes (tels que l'éthylène, le propylène, les différents butènes, ou encore les pentènes, par exemple) sont utilisés dans l'industrie des plastiques, par exemple pour réaliser du polypropylène ou du polyéthylène, ainsi que dans d'autres domaines de l'industrie chimique et de celle des carburants.
L'éthylène, l'alcène le plus simple, tient une place centrale dans la chimie organique industrielle : c'est le composé organique le plus produit au monde. Il permet notamment de générer le polyéthylène, un plastique de premier plan. Par réaction (d'oxydation, d'halogénation), on peut aussi transformer l'éthylène en de nombreux produits utiles à l'industrie.
Le propylène tient un rôle d'importance comparable : on en dérive par polymérisation une matière plastique, le polypropylène. Les caractéristiques techniques de ce produit, en termes de résistance, de densité, de solidité, de déformabilité, de transparence n'ont pas d'équivalents. Le marché mondial du polypropylène se développe constamment depuis son invention en 1954.
Le butylène existe sous quatre formes dont l'une, l'iso-butylène, entre dans la composition du méthyl-tertio-butyl-ether-(MTBE), un additif antidétonant pour les carburants automobiles. L'iso-butylène peut également être utilisé pour générer de l'iso-octène, que l'on peut ensuite réduire en iso-octane (2,2,4-triméthyl-pentane); le ratio combustion/explosion très élevé de l'iso-octane en fait le meilleur carburant pour les moteurs dits « à essence ».
L'amylène, l'hexène et l'heptène existent sous de nombreuses formes selon l'emplacement et la configuration de la double chaîne. Ces produits connaissent des applications industrielles réelles, mais de moindre importance que l'éthylène, le propylène, ou les butènes.
Tous ces alcènes sont actuellement produits par cracking catalytique des produits pétroliers (ou par un dérivé du procédé Fisher-Tropsch dans le cas de l'hexène, à partir de charbon ou de gaz). Leur coût est donc naturellement indexé sur celui du pétrole. Par ailleurs, le cracking catalytique présente parfois des difficultés techniques importantes, qui se traduisent en complexité du procédé et en coût de production.

Indépendamment des considérations précédentes, la bioproduction des matières plastiques (« bioplastiques ») connaît un essor important. La motivation vient de considérations économiques liées au coût du pétrole, et de considérations environnementales globales (produits neutres en carbone) ou locale (gestion des déchets).
La principale famille de bioplastiques est celle des polyhydroxyalcanoates (PHA). Il s'agit de polymères obtenus par condensation de molécules comprenant à la fois un groupement acide et un groupement alcool. La condensation a lieu par estérification de l'acide sur l'alcool du monomère suivant. Ce lien ester n'est pas aussi stable que le lien direct carbone-carbone que l'on retrouve dans les polymères de plastique classiques, ce qui explique la biodégradabilité, en quelques semaines à quelques mois, des PHA.
Dans la famille des PHA, on peut notamment citer le poly-3-hydroxy-butyrate (PHB), un polymère du 3-hydroxy-butyrate, ou le poly-hydroxy-butyrate-valérate (PHBV), un polymère alternant le 3-hydroxy-butyrate et le 3-hydroxy-valérate.
Le PHB est produit naturellement par des souches bactériennes particulières, telles que *Alcaligenes eutrophus* ou *Bacillus megaterium.* Des bactéries de laboratoire, telles *qu'Escherichia coli,* ayant intégré des voies de synthèses menant au PHB ou aux PHA en général, ont été construites. Le composé ou son polymère peut représenter, dans certaines conditions de laboratoire, jusqu'à 80% de la masse de la bactérie. (Wong MS et al, Biotech. Bioeng 2008). Des tentatives de production industrielle du PHB ont été menées dans les années 1980, mais le coût de fabrication par fermentation était considéré alors comme trop élevé. Des projets de production directe de ces composés dans des plantes génétiquement modifiées (ayant intégré les enzymes clé de la voie de synthèse de PHB des bactéries productrices) sont en cours. Ils pourraient être associés à de meilleurs coûts d'exploitation.

La génération par voie biologique d'alcanes ou d'autres molécules organiques utilisables comme carburants ou comme précurseurs de résines synthétiques s'impose dans le contexte d'une exploitation industrielle durable en harmonie avec les cycles géochimiques. La première génération de biocarburants a consisté en la production fermentative d'éthanol, les procédés de fermentation et de distillation étant déjà en place dans l'industrie agroalimentaire. La production de biocarburants de seconde génération est dans une phase exploratoire, couvrant notamment la production d'alcools à chaînes longues (butanol et pentanol), de terpènes, d'alcanes linéaires et d'acides gras. Deux revues récentes dressent un tableau général des recherches dans ce domaine : Ladygina N et al., Process Biochemistry 2006 41 :1001, et Wackett LP Current Opinion in Chemical Biology, 2008, 21 :187.

Dans la famille chimique des alcènes, l'isoprène (2-méthyl-1,3-butadiène) est le motif terpénique qui conduit par polymérisation au caoutchouc. D'autres terpènes pourraient être développés, par voie chimique, biologique ou mixte, en produits utilisables comme biocarburants ou pour fabriquer des plastiques. Des publications récentes montrent que la voie du mévalonate (un intermédiaire clé dans la biosynthèse des stéroïdes chez de nombreux organismes) pourrait être utilisée de façon à produire efficacement des produits de la famille des terpènes avec des rendements industriels (Withers ST et al., Appl. Environ Microbiol 2007 73 :6277).

La production d'alcènes terminaux (éthylène mono- ou bi-substitué en position 2, H2C=C(R¹)(R²) a été apparemment moins explorée : la production d'iso-butylène à partir d'iso-valérate par la levure *Rhodotorula minuta,* a été détectée (Fujii T. et al, Appl. Environ. Microbiol. 1988 54:583), mais l'efficacité de cette conversion, inférieure à 1 millionième par minute, soit environ 1 pour 1000 par jour, est loin de permettre une application industrielle. Le mécanisme réactionnel a été élucidé dans Fukuda H et al., BBRC 1994 201 :2 :516 : il fait intervenir une enzyme de la famille des cytochromes P450 qui effectue une réaction de décarboxylation de l'iso-valérate, par réduction d'un groupe oxoferryle Fe^{v}=O. A aucun moment la réaction ne fait intervenir l'hydroxylation de l'iso-valérate. L'isovalérate est par ailleurs un composé intermédiaire du catabolisme de la leucine. La biosynthèse massive d'iso-butylène par cette voie paraît très défavorable, puisqu'il faudrait synthétiser et dégrader une molécule de leucine pour former une molécule d'iso-butylène. Enfin, l'enzyme catalysant la réaction emploie un hème comme co-facteur, se prêtant mal à l'expression recombinante chez les bactéries et à l'amélioration des paramètres enzymatiques. Pour toutes ces raisons, il paraît peu probable que cette voie de l'art antérieur puisse servir de base à un déploiement industriel. D'autres micro-organismes ont été mentionnés comme marginalement capables de générer naturellement de l'iso-butylène à partir d'iso-valérate. Les rendements obtenus sont encore plus faibles que ceux obtenus en utilisant *Rhodotorula minuta* (Fukuda H. et al, Agric. Biol. Chem. 1984 48 :1679).

Ces mêmes travaux font également état de la production naturelle de propylène : de nombreux micro-organismes peuvent produire du propylène, avec un rendement là encore extrêmement faible.

La production d'éthylène par les plantes est décrite depuis longtemps (Meigh et al, 1960 Nature 186 :902). La méthionine constitue le précurseur de l'éthylène suivant la voie métabolique élucidée (Adams et Yang, PNAS 1979 76 :170). Une conversion à partir du 2-oxoglutarate a également été rapportée (Ladygina N et al., Process Biochemistry 2006 41 :1001). Puisqu'une seule molécule d'éthylène nécessite la production préalable d'une chaîne de quatre ou cinq atomes de carbone, les bilans matériel et énergétique de toutes ces voies sont défavorables et augurent mal de leur application industrielle pour la bioproduction d'alcènes.
Antérieurement à la caractérisation des étapes enzymatiques qui, chez les plantes, convertissent en méthylène son véritable précurseur métabolique, la S-adénosyl-méthionine (SAM), via la formation d'1-amino-cyclopropane-1-carboxylate (ACC) (Adams et Yang, PNAS 1979 76 :170 ), plusieurs autres hypothèses ont été proposées dans la littérature scientifique pour expliquer la production d'éthylène, parmi lesquelles la décarboxylation de l'acrylate (H2C=CH-CO2H) lequel proviendrait de la déshydratation du 3-hydroxy-propionate. Plusieurs articles spéculent explicitement sur la voie métabolique qui convertirait en éthylène, via l'acrylate, le 3-hydroxy-propionate, pour interpréter des expériences de traçage radio-actif de l'éthylène en fournissant des substrats marqués au ¹⁴C à des préparations de tissus végétaux, la béta-alanine-2-¹⁴C à des extraits de cotylédon de haricot (Stinson and Spencer, Plant Physiol., 1969, 44 :1217 ; Thompson and Spencer, Nature 1966, 210 :5036), et le propionate-2-¹⁴C à des homogénats de pulpe de banane. (Shimokawa et Kasai, Agr. Biol. Chem. 1970 34 :11 :1640). Toutes ces spéculations sur l'mplication de 3-hydroxypropionate et d'acrylate dans la production métabolique d'éthylène, qui n'ont pas conduit à la caractérisation d'activités enzymatiques, ont disparu de la littérature scientifique dès que le rôle de la méthionine, du SAM et de l'ACC a été découvert (Hanson et Kende, Plant Physiology 1976 57 :528 ; Adams et Yang, PNAS 1979 76 :170).

Il n'existe donc actuellement pas, à ma connaissance, de procédé efficace pour produire par synthèse microbiologique des alcènes terminaux tels que l'éthylène, le propylène, le 1-butylène, l'iso-butylène, le 1-amylène ou l'iso-amylène. Un tel procédé permettrait d'éviter de consommer des produits pétroliers, et de réduire les coûts de production de matière plastique et de carburant. Enfin, il pourrait éventuellement avoir un effet global important sur l'environnement en permettant de stocker du carbone sous forme solide.

### Résumé de invention

La présente invention décrit un procédé permettant de réaliser la synthèse de composés alcènes par voie biologique.

L'invention découle de la conception d'une nouvelle voie de synthèse de composés alcène terminaux basée sur la conversion de 3-hydroxy-alcanoates de la formule

HO-CO-CH₂-C(R¹)(R²)-OH

dans laquelle R¹ et R² sont choisis, indépendamment, parmi un atome d'hydrogène, un résidu méthyle et un résidu éthyle. L'invention est également basée sur la démonstration que cette conversion peut être réalisée par voie biologique, en utilisant une enzyme de type mévalonate diphosphate décarboxylase ou des variants de celle-ci. L'invention peut être mise en oeuvre *in vitro,* dans des
systèmes acellulaires, ou en utilisant des micro-organismes. L'invention vise également la production des alcènes à partir d'une source de carbone, et notamment un hydrate de carbone (notamment le glucose), un polyol (notamment le glycérol), un polymère biodégradable (notamment l'amidon, la cellulose, le poly-3-hydroxy-alcanoate) ; la source de carbone étant convertie par un micro-organisme en un intermédiare métabolique de la famille des 3-hydroxy-alcanoates, qui sera ensuite converti en alcène terminal.

Plus précisément, un objet de invention réside dans un procédé de production d'un alcène terminal, caractérisé en ce qu'il comprend une étape de conversion d'un 3-hydroxy-alcanoate par une enzyme présentant une activité mévalonate diphosphate décarboxylase, dans lequel le 3-hydroxy-alcanoate est un composé de la formule HO-CO-CH₂-C(R¹)(R²)-OH ou O⁻-CO-CH₂-C(R¹)(R²)-OH dans laquelle R¹ et R² sont choisis, indépendamment, parmi un atome d'hydrogène, un résidu méthyle et un résidu éthyle, et dans lequel la conversion se fait en présence d'un co-substrat, ledit co-substrat étant l'ATP, un rNTP, un dNTP ou un mélange de plusieurs de ces molécules, un polyphosphate, ou le pyrophosphate.

En conséquence, la présente invention se rapporte à l'utilisation de composés 3-hydroxy-alcanoate, à titre de composé précurseur ou substrat, pour la production de composés alcènes terminaux.

Dans des modes de réalisation particuliers de l'invention :
- on convertit le 3-hydroxy-propionate en méthylène; ou
- on convertit le 3-hydroxy-butynate en propylène ; ou
- on convertit le 3-hydroxy-valérate en 1-butylène ; ou
- on convertit le 3-hydroxy-3-methyl-butyrate (ou 3-hydroxy-isovalérate) en iso-butylène ; ou
- on convertit le 3-hydroxy-3-methyl-valérate en iso-amylène.

L'invention concerne encore l'utilisation d'une enzyme mévalonate diphosphate décarboxylase, ou d'un micro-organisme produisant une mévalonate diphosphate decarboxylase, pour la production de composés alcènes terminaux à partir de 3-hydroxy-alcanoates comme définis ci-dessus.

L'invention concerne également une composition comprenant un micro-organisme produisant une mévalonate diphosphate décarboxylase, un milieu de culture approprié et un composé 3-hydroxy-alcanoate comme défini ci-dessus.

L'enzyme ayant une activité mévalonate diphosphate décarboxylase peut être une enzyme comprenant tout ou partie de la séquence SEQ ID NO: 6 ou une enzyme présentant une homologie de séquence d'au moins 15% avec celle-ci.

L'invention concerne aussi l'utilisation d'une enzyme ayant une activité mévalonate diphosphate décarboxylase et comprenant tout ou partie de la séquence SEQ ID NO: 6, ou d'une enzyme présentant une homologie de séquence d'au moins 15% avec celle-ci, pour produire un alcène terminal comme défini ci-dessus.

Il est aussi décrit un procédé de production d'une enzyme ayant une activité mévalonate diphosphate décarboxylase et comprenant tout ou partie de la séquence SEQ ID NO: 6 ou d'une enzyme présentant une homologie de séquence d'au moins 15% avec celle-ci, le procédé comprenant la culture d'un microorganisme comprenant un acide nucléique recombinant codant ladite séquence dans des conditions permettant l'expression de cette séquence.

Il est aussi décrit un microorganisme comprenant un acide nucléique recominant codant une enzyme ayant une activité décarboxylase et comprenant tout ou partie de la séquence SEQ ID NO : 6 ou une enzyme présentant une homologie de séquence d'au moins 15% avec celle-ci.

### Définitions

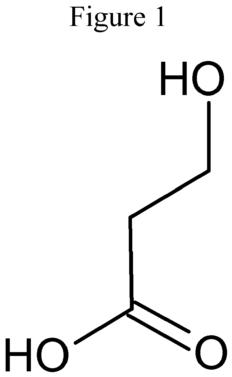

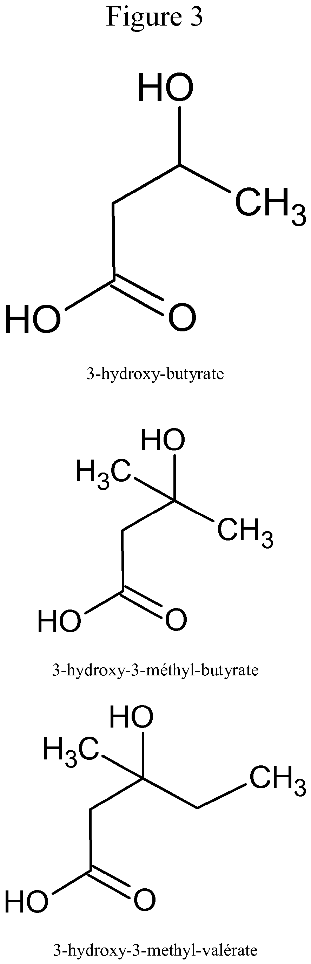

Par "3-hydroxy-alcanoate", on entend des molécules comprenant comme motif commun le 3-hydroxy-propionate (figure 1), et éventuellement une ou deux substitutions alcoyles sur le carbone 3. Ces résidus ou radicaux alcoyles peuvent être linéaires ou branchés. Dans la présente demande, les termes « alcoyle » et « alkyle ont la même signification et sont interchangeables. De même, les termes « résidu » et « radical » ont la même signification et sont interchangeables. Les résidus méthyle, éthyle, propyle, iso-propyle, butyle, iso-butyle sont des exemples de ces résidus alcoyles. Le carbone 3 devient un centre chiral si les deux substitutions alcoyles sont différentes. La présente définition englobe les deux formes chirales, même si l'une des deux formés, par exemple la forme R, est la principale forme produite naturellement. Des exemples de 3-hydroxy-alcanoates sont exposés en figure 3. Eventuellement, des substitutions alcoyles peuvent être ajoutées sur le carbone 2, qui est alors susceptible lui aussi de devenir chiral (si les deux substituants sont différents). Indifféremment, les configurations des substrats 3-hydroxy alcanoates dans la présente invention recouvrent l'ensemble des stéréo-isomères. Les 3-hydroxy-alcanoates correspondent soit au 3-hydroxy-proplonate soit à des variantes ou dérivés du 3-hydroxy-proplonate dans lesquels un des deux ou les deux atomes d'hydrogène portés par le carbone 3 sont substitués par un motif composé seulement d'atomes de carbone et d'hydrogène, le nombre de carbone de ces substituants variant de 1 à 5, de préférence de 1 à 3, tel que le méthyle, l'éthyle, le propyle, l'isopropyle, le butyle ou isobutyle. Le suffixe « oate » peut signifier ici indifféremment soit l'ion carboxylate (COO-) soit l'acide carboxylique (COOH). Il n'est pas utilisé pour désigner un ester.

Les 3-hydroxy-alcanoates utilisés dans le procédé selon l'invention sont des composés de formule suivante: HO-CO-CH₂-C(R¹)(R²)-OH ou O-CO-CH₂-C(R¹)(R²)-OH dans laquelle R¹ et R² sont choisis, indépendamment, parmi un atome d'hydrogène, un résidu méthyle et un résidu éthyle.

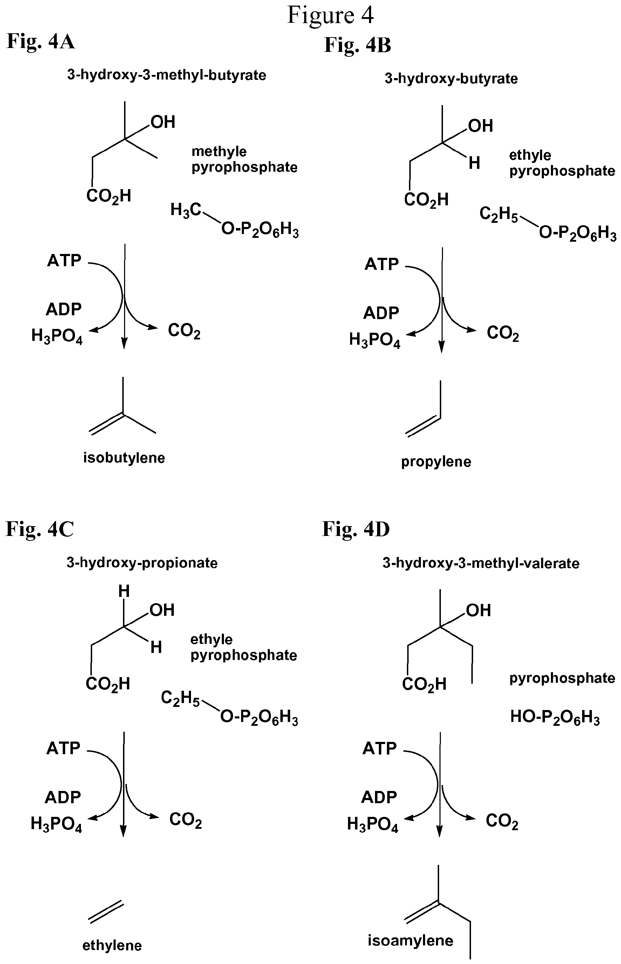

Par « alcènes terminaux», on entend au sens de la présente invention les molécules composées seulement de carbone et d'hydrogène (hydrocarbures insaturés de formule CnH2n) comprenant l'éthylène et les molécules organiques dérivant de l'éthylène par mono- ou bi-substitution des deux atomes d'hydrogène liés au carbone 2 par des radicaux alcoyles, linéaires ou branchés. Les alcènes terminaux répondent à la formule H2C=C(R¹)(R²) dans laquelle R¹ et R² sont choisis, indépendamment, parmi un atome d'hydrogène et un radical alcoyle, linéaire ou ramifié. avant par exemple de 1 à 4 atomes de carbone, ou de 1 à 3 atomes de carbone. L'un au moins des deux substituants du carbone 2 de l'alcène peut être un radical alcoyle linéaire ou ramifié. Les alcènes terminaux comprennent les composés ramifiés iso-alcènes, comme par exemple l'iso-butylène. Un alcène terminal qui est le produit d'un procédé selon l'invention est un composé de formule H2C=C(R¹)(R²) dans laquelle R¹ et R² sont choisis, indépendamment, parmi un atome d'hydrogène, un résidue méthyle et un résidu éthyle. Des exemples préférés de composés alcènes terminaux selon
l'invention sont notamment, l'éthylène, le propylène, l'iso-butylène, et l'iso-amylène (figure 4), ou encore le 1-butylène et le 1-amylène.

« Source de carbone » désigne ici tout composé carboné utilisable comme substrat pour les organismes selon l'invention. Ce terme englobe le glucose ou tout autre hexose, le xylose ou tout autre pentose, des polyols comme le glycérol, le sorbitol ou le mannitol, ou encore des polymères tels que l'amidon, la cellulose ou l'hémicellulose, ou encore des poly-3-hydroxy-alcanoates comme le poly-3-hydroxy-butyrate. Il peut s'agir de tout substrat permettant la croissance de micro-organismes, comme le formate par exemple. Il peut également s'agir de CO₂ dans le cas où l'organisme est susceptible de réaliser de la photosynthèse.

Par « recombinant », on désigne ici la modification génétique artificielle d'un organisme, soit par addition, soustraction, ou modification d'un gène chromosomique ou extra-chromosomique ou d'un motif de régulation tel qu'un promoteur, soit par fusion d'organismes, soit par addition d'un vecteur de toute nature, par exemple plasmidique. L' « expression recombinante » désigne la production d'une protéine impliquant une modification génétique, préférablement pour produire une protéine d'origine étrangère ou hétérologue vis-à-vis de son hôte, c'est-à-dire qui ne survient pas naturellement dans l'hôte de production, ou pour produire une protéine endogène modifiée ou mutée.

Par « surexpression », ou « surexprimant », on désigne ici l'expression recombinante d'une protéine, de préférence issue d'un organisme différent de celui dans lequel elle est exprimée, augmentée de 10% au moins et de préférence de 20%, 50%, 100%, 500% ou éventuellement plus par rapport à l'expression naturelle de ladite protéine. Entre également dans le cadre de cette définition le cas où il n'y a pas d'expression naturelle de ladite protéine.

Un « co-substrat » est un produit ajouté à la réaction enzymatique, de façon à en améliorer certains paramètres et en premier lieu son activité, ledit produit et le substrat principal étant consommés en quantités égales. Le co-substrat doit donc être ajouté à la réaction en concentration comparable à celle du substrat principal. Selon l'enzyme, la présence d'un co-substrat peut être nécessaire à la réaction enzymatique.

Un « co-facteur » est un produit ajouté à la réaction enzymatique, de façon à en améliorer certains paramètres et en premier lieu son activité, ledit produit n'étant pas consommé au cours de la réaction, et nécessitant donc de n'être ajouté qu'en concentration faible, proportionnelle à la quantité d'enzyme, cette concentration étant donc dite « catalytique ».

Une « partie » d'une séquence d'acides aminés désigne un fragment comprenant au moins 10, de préférence au moins 20, 30, 40 ou 50 résidus d'acides aminés consécutifs de ladite séquence.

Par « homologie », on entend l'existence d'une similarité entre deux séquences telle qu'elle est mesurée par le pourcentage d'identité entre lesdites deux séquences.

Les composés chimiques sont souvent connus sous plusieurs noms, officiels ou d'usage. Ici, les noms habituels des molécules ont été préférés. Ainsi :
- « éthylène » est utilisé pour désigner l'éthène
- « propylène » est utilisé pour désigner le propène
- « butylène » est utilisé pour désigner le butène
- « isobutylène » est utilisé pour désigner le 2-méthyle-propène ou isobutène
- « amylène » est utilisé pour désigner le pentène
- « isoamylène » est utilisé pour désigner le 2-méthyle-but-1-ène ou isopentène
- « propionate » est utilisé pour désigner l'acide propanoïque ou l'ion propanoate
- « butyrate » est utilisé pour désigner l'acide butanoïque ou l'ion butanoate
- « valérate » est utilisé pour désigner l'acide pentanoïque ou l'ion pentanoate

### Description détaillée de invention

L'invention décrit notamment un procédé de production d'alcènes terminaux comprenant une étape de décarboxylation enzymatique de composés 3-hydroxy-alcanoates comme définis ci-dessus. L'invention découle également de l'utilisation de décarboxylases de type mévalonate diphosphate décarboxylase pour catalyser cette réaction, et de substrats tels que le 3-hydroxy-butyrate, le 3-hydroxy-valérate, le 3-hydroxy-3-méthyl-butyrate (ou 3-hydroxy-isovalérate) et le 3-hydroxy-propionate. L'invention décrit l'utilisation de co-facteurs, dont l'éthyle diphosphate, le propyle diphosphate, le méthyle diphosphate, des analogues de ces molécules, et le pyrophosphate. L'invention décrit encore l'utilisation de co-substrats, tel que l'ATP ou d'autres composés comportant une liaison phospho-anhydride comme définis ci-dessus.

L'invention porte aussi sur l'utilisation de sources de carbone, telles que le glucose, pour produire directement les alcènes terminaux à partir de cellules entières, la voie de synthèse passant par des 3-hydroxy-alcanoates comme définis ci-dessus.

L'invention porte aussi sur des microorganismes ou des plantes, naturels ou modifié, produisant de façon endogène un 3-hydroxy-alcanoate comme défini ci-dessus, et exprimant par ailleurs une mévalonate diphosphate décarboxylase transformant lesdits 3-hydroxy-alcanoates en alcènes terminaux. Les composés alcènes produits, notamment le propylène, l'éthylène et l'iso-butylène, sont des molécules clés dans l'industrie des plastiques et des carburants, et leur fabrication industrielle par voie biologique, à partir de ressources renouvelables, constitue une innovation majeure.

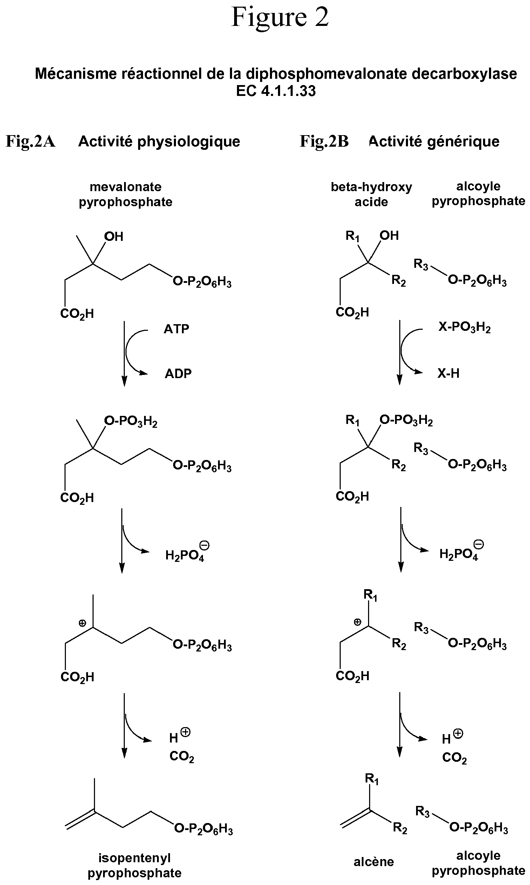

L'invention découle ainsi de la conception d'une nouvelle voie de synthèse de composés de type alcène terminal basée sur la conversion de composés de type 3-hydroxy-alcanoate comme définis ci-dessus. L'invention démontre que cette conversion peut être réalisée par voie biologique, en utilisant une enzyme de type mévalonate diphosphate décarboxylase, qui permet de transformer un 3-hydroxy-alcanoate comme défini ci-dessus en alcène terminal. Comme illustré dans la figure 2, cette conversion se fait via un intermédiaire réactionnel de structure 3-phospho-hydroxy-alcanoate.

L'étape de conversion selon l'invention peut être réalisée *in vitro,* en présence d'une enzyme isolée (ou d'un système enzymatique comprenant en outre un ou plusieurs co-facteurs) ou en culture, en présence d'un micro-organisme produisant l'enzyme.

Comme décrit ici dans l'exemple 5, un rapport signal sur bruit de fond (mesuré en absence d'enzyme) du taux de conversion d'un facteur 100 environ a pu être observé dans certaines conditions. L'affinité vis-à-vis du 3-hydroxy-isovalerate (HIV) a été mesuré de l'ordre de 40 mM. Il n'était pas évident qu'une telle activité enzymatique, très significative, ait pu être obtenue : en effet, il est bien connu des biochimistes considérant la théorie et la pratique de l'enzymologie que les sites actifs des enzymes contiennent des éléments structurels permettant la reconnaissance, la liaison et la conversion chimique de certains substrats spécifiques. La littérature scientifique regorge de données expérimentales indiquant que les modifications de taille ou de charge électrique, même mineures, sont des critères d'exclusion pour les substrats. Spécifiquement, aucune prédiction scientifique ne pouvait permettre de prévoir que les MDP-décarboxylases pourraient utiliser des molécules de type 3-hydroxy-alcanoate comme substrat en général, et en particulier le 3-hydroxy-isovalerate, celui-ci différant du mévalonate-diphosphate non seulement par la taille (MW de 118 contre 308 pour le mévalonate diphosphate) mais également par les charges électriques du groupe diphosphate présent sur le substrat naturel, le mévalonate-diphosphate.

Dans un mode particulier, un co-facteur est ajouté à la réaction de façon à réaliser une complémentation stérique ou électronique dans la gorge catalytique. Le co-facteur est avantageusement choisi parmi l'ion pyrophosphate, le méthyl-diphosphate, l'éthyl-diphosphate, ou le propyl-diphosphate. Plus généralement, le co-facteur est un composé contenant le motif phosphoanhydride, de formule générale R-O-PO₂H-O-PO₃H₂ dans laquelle R est notamment un atome d'hydrogène, un résidu alcoyle, linéaire, branché ou cyclique, de préférence de 1 à 10 ou de 1 à 5 atomes de carbone, ou tout autre radical organique monovalent. Les motifs analogues correspondant aux mono-esters du methylène diphosphonate, de formule générale R-O-PO₂H-CH₂-PO₃H₂, dans lesquels le phosphoanhydride est remplacé par un pont méthylène présentant l'avantage de ne pas s'hydrolyser, font également partie de l'invention.

La conversion se fait en présence d'un co-substrat, ledit co-substrat étant l'ATP, un rNTP, un dNTP ou un mélange de plusieurs de ces molécules, un polyphosphate, ou le pyrophosphate. Le co-substrat est généralement présent dans l'hôte. Cependant, dans un autre mode particulier, un co-substrat peut être ajouté à la réaction, choisi parmi l'ATP, un rNTP, un dNTP, un mélange de plusieurs rNTP ou dNTP, un polyphosphate, et de préférence le pyrophosphate.

Dans un mode de mise en oeuvre particulier de l'invention, on utilise un micro-organisme produisant la mévalonate diphosphate décarboxylase. Dans un mode de réalisation préféré, le microorganisme est recombinant dans le sens où il produit une mévalonate diphosphate décarboxylase hétérologue par rapport à l'hôte de production. Le procédé peut ainsi être réalisé directement dans le milieu de culture, sans qu'il soit nécessaire de séparer ou de purifier le système enzymatique. De manière particulièrement avantageuse, on utilise un micro-organisme ayant la propriété naturelle ou artificielle de produire un ou plusieurs 3-hydroxy-alcanoates comme définis ci-dessus de façon endogène, et exprimant ou surexprimant par ailleurs une mévalonate diphosphate décarboxylase, naturelle ou modifiée, afin de produire des alcènes terminaux directement à partir d'une source de carbone présente en solution.

Les micro-organismes mis en oeuvre dans l'invention peuvent être des procaryotes ou des eucaryotes, et notamment des bactéries, levures, cellules végétales, champignons et moisissures, cellules animales. Dans un mode particulier, les micro-organismes sont des bactéries, notamment de souche *Alcaligenes eutrophus* ou *Bacillus megaterium.*

Dans un autre mode particulier, les micro-organismes sont des bactéries recombinantes de souche *Escherichia coli* ayant été modifiées de façon à produire de façon endogène un ou des 3-hydroxy-alcanoates comme définis ci-dessus, et convertissant ceux-ci en alcènes terminaux.

Dans un autre mode particulier les micro-organismes sont des levures recombinantes, produisant des 3-hydroxy-alcanoates comme définis ci-dessus, et convertissant ceux-ci en alcènes terminaux.

Dans un autre mode particulier, on utilise un micro-organisme producteur d'un ou plusieurs 3-hydroxy-alcanoates comme définis ci-dessus d'une part, et une mévalonate diphosphate décarboxylase, éventuellement exprimée par un second micro-organisme, d'autre part. Eventuellement, on cultive et on utilise concomitamment les deux microorganismes dans le procédé selon l'invention.

Dans un autre mode particulier, des plantes entières exprimant une mévalonate diphosphate décarboxylase, éventuellement modifiés par transgénèse, sont utilisés pour produire des alcènes terminaux à partir de 3-hydroxy-alcanoates comme définis ci-dessus, que ceux-ci soient produits de manière endogène ou apportés de manière exogène.

Dans un autre mode particulier de mise en oeuvre, on utilise un micro-organisme photosynthétique ayant la propriété naturelle ou artificielle de produire un ou plusieurs 3-hydroxy-alcanoates comme définis ci-dessus de façon endogène, et surexprimant par ailleurs une mévalonate diphosphate décarboxylase, naturelle ou modifiée, afin de produire des alcènes terminaux directement, à partir de CO₂ présent en solution. De préférence, le micro-organisme est une bactérie photosynthétique, ou une micro-algue.

Les plantes et les micro-organismes décrits ci-dessus représentent d'autres objets de la présente invention, de même que leur utilisation pour la production de composés alcènes terminaux.

Comme il sera décrit dans la suite, le procédé de l'invention peut être mené en micro-aérophilie.

Par ailleurs, dans un mode.de réalisation préféré, le procédé est réalisé en présence d'un système de collecte gazeuse des alcènes terminaux dégazant de la réaction.

La décarboxylase utilisée dans un procédé de l'invention est un représentant de la superfamille phylogénétique de la mévalonate-diphosphate (MDP) décarboxylase (nomenclature enzymatique EC 4.1.1.33), c'est-à-dire une enzyme, naturelle ou artificielle, spécifiée par un gène natif ou synthétique, éventuellement capable de catalyser la réaction présentée en figure 2. Comme illustré dans la figure 2, le procédé de l'invention se fait via un intermédiaire réactionnel 3-phospho-hydroxy-alcanoate, et l'enzyme mise en oeuvre présente une activité décarboxylase et une activité phosphorylase.

La MDP décarboxylase est une enzyme inpliquée dans la biosynthèse du cholestérol. Cette enzyme a été isolée à partir d'organismes divers tels que les animaux, les champignons, les levures, et certaines bactéries. Elle pourrait également être exprimée par certaines plantes (Lalitha et al, 1985). De nombreux gènes spécifiant cette enzyme ont été clonés et séquencés. Ces enzymes sont généralement composées de 300 à 400 acides-aminés, et font intervenir de l'ATP comme co-substrat, qui est transformé au cours de la réaction en ADP et en phosphate inorganique. Le groupement phosphate est transféré de la molécule d'ATP à l'alcool tertiaire du mévalonate diphosphate en libérant de l'ADP. L'intermédiaire réactionnel phosphorylé sur le groupe 3-hydroxyle subit ensuite une élimination du groupe phosphate, libérant dans le cas physiologique l'isopentenyl-pyrophosphate (figure 2).

La structure tridimensionnelle de plusieurs enzymes de cette famille a été résolue. Relativement peu de travaux ont été menés à ce jour sur les enzymes de cette famille, qui n'ont été étudiées que dans le cadre de la description précise de la voie de biosynthèse du cholestérol. En revanche, à ma connaissance, aucune étude n'a encore été conduite pour détourner cette enzyme de sa fonction naturelle et en faire un catalyseur industriel.

Plusieurs exemples de MDP décarboxylases provenant de différents organismes sont donnés dans les séquences SEQ ID NO : 1 à SEQ ID NO: 16.

Ainsi, dans un mode de réalisation préféré, l'enzyme mise en oeuvre est une décarboxylase, comprenant de préférence une séquence en acides aminés choisie parmi SEQ ID NOs : 1, 2, 3, 4, 5, 6, 7, 8, 9, 10, 11, 12, 13, 14, 15 ou 16 ou une séquence présentant au moins 15% d'homologie de séquence avec l'une de celles-ci et conservant une activité décarboxylase. Des enzymes préférées comportent avantageusement au moins 50% d'homologie de séquence, préférentiellement au moins 80%, plus préférentiellement au moins 85%, encore plus préférentiellement, au moins 90, 95, 96, 97, 98 ou 99% d'homologie de séquence avec l'une des séquences primaires SEQ ID NO : 1, 2, 3, 4, 5, 6, 7, 8, 9, 10, 11, 12, 13, 14, 15 et 16. Le degré d'homologie de séquence peut être déterminé par différentes méthodes et au moyen de logiciels connus de l'homme du métier, comme par exemple selon la méthode CLUSTAL ou les logiciels BLAST ou dérivés, ou en utilisant un algorithme de comparaison de séquences tel que celui de Needleman et Wunsch (J. Mol. Biol. 1970 48 :443) ou de Smith et Waterman (J. Mol. Biol. 1981 147 :195).

Une décarboxylase préférée de l'invention est représentée par l'enzyme de séquence SEQ ID NO : 6, ainsi que toute enzyme présentant une homologie de séquence significative avec celle-ci. Des enzymes préférées comportent avantageusement au moins 50% d'homologie de séquence, préférentiellement au moins 80%, plus préférentiellement au moins 85%, encore plus préférentiellement, au moins 90, 95, 96, 97, 98 ou 99% d'homologie de séquence avec la séquence primaire SEQ ID NO : 6. Cette enzyme a été clonée à partir de *Picrophilus torridus* et produite par voie recombinante dans le cadre de la présente invention. Comme illustré dans les exemples, cette enzyme est particulièrement efficace pour produire des composés alcènes terminaux selon la présente invention. Notamment, un objet de l'invention réside dans l'utilisation d'une enzyme décarboxylase comprenant tout ou partie de SEQ ID NO:6 ou d'une enzyme présentant une homologie de séquence significative et de préférence d'au moins 15% avec SEQ ID NO:6, pour la production de composés alcènes terminaux à partir de 3-hydroxy-alcanoates comme définis ci-dessus. Par homologie de séquence
significative, on entend une homologie de séquence détectable en utilisant les algorithmes précédemment cités, et de préférence une homologie de séquence supérieure à 15%. Les organismes phylogénétiquement les plus proches de *Picrophilus torridus,* tels que *Ferroplasma acidarmanus, Thermoplasma acidophilum, Thermoplasma volcanium* et *Picrophilus oshimae* sont susceptibles de produire les MDP décarboxylases les plus proches de celle de la SEQ ID NO 6. L'homologie de séquence vis-à-vis de la SEQ ID NO 6 de la MDP décarboxylase de *Thermoplasma acidophilum* (AC number Q9HIN1) est ainsi de 38% ; celle de *Thermoplasma volcanium* (Q97BY2) est homologue à la SEQ ID NO 6 à 42% environ. L'utilisation de ces MDP décarboxylases est tout particulièrement envisagée dans la présente invention.

D'autres enzymes de type mévalonate diphosphate décarboxylase, naturelles ou synthétiques, peuvent être sélectionnées pour
leur capacité à produire des alcènes terminaux selon l'invention. Ainsi, un test de sélection comprend la mise en contact de l'enzyme purifiée, ou d'un microorganisme produisant l'enzyme, avec le substrat de la réaction et la mesure de la production du composé alcène terminal. Des exemples de tels tests sont fournis dans la partie expérimentale, qui expose des tests sur plus de 60 enzymes différentes.

L'enzyme mise en oeuvre peut être toute mévalonate diphosphate décarboxylase naturelle ou produite ou optimisée de manière artificielle. En particulier, on utilisé avantageusement une mévalonate diphosphate décarboxylase présentant une activité optimisée vis-à-vis d'un ou de plusieurs 3-hydroxy-alcanoates comme définis ci-dessus.
L'enzyme peut être produite ou sélectionnée, à partir d'une mévalonate diphosphate décarboxylase de référence (naturelle ou
elle-même déjà synthétique ou optimisée), par des techniques d'ingénierie des protéines telles que la mutagenèse aléatoire, la mutagenèse massive, la mutagenèse dirigée, le DNA-shuffling, le shuffiling synthétique, l'évolution *in vivo,* ou la synthèse complète de gènes.
À cet égard, il est également décrit un procédé de préparation d'une enzyme ayant une activité mévalonate diphosphate décarboxylase vis-à-vis d'un substrat 3-hydroxy-alcanoate comme défini ci-dessus, le procédé comprenant une étape de traitement d'une source d'enzyme et la sélection d'une enzyme ayant des propriétés accrues vis-à-vis dudit substrat, par comparaison à l'enzyme non traitée.
L'enzyme utilisée dans l'invention peut ainsi être naturelle ou synthétique, et produite par voie chimique, biologique, ou génétique. Elle peut également être modifiée chimiquement, par exemple pour améliorer son activité, sa résistance, sa spécificité, sa purification, ou pour l'immobiliser sur support.
L'invention est caractérisée par l'utilisation d'une mévalonate diphosphate décarboxylase, en particulier d'une MDP décarboxylase naturelle ou modifiée, pour convertir des 3-hydroxy-alcanoates comme définis ci-dessus en alcènes terminaux.
Le substrat naturel de la MDP décarboxylase est le mévalonate diphosphate, qui n'entre pas dans la définition des 3-hydroxy-alcanoates.

La réaction générique réalisée par la MDP carboxylase en utilisant divers 3-hydroxy-alcanoates est représentée dans la figure 2B. On conçoit que ces réactions mènent directement et en une seule étape à des alcènes terminaux.

Dans un premier mode de réalisation, on utilise de l'enzyme native ou recombinante, purifiée ou non, pour transformer un 3-hydroxy-alcanoate comme défini ci-dessus en alcène terminal. Pour ce faire, on incube la préparation
d'enzyme en présence du substrat dans des conditions physico-chimiques permettant à l'enzyme d'être active, et on laisse l'incubation se faire pendant un temps suffisant. A l'issue de l'incubation, on mesure éventuellement la présence de l'alcène terminal en utilisant tout système de détection connu de l'homme du métier comme la chromatographie en phase gazeuse ou des tests colorimétriques permettant de mesurer l'apparition du produit alcène, de phosphate libre, ou encore permettant de mesurer la disparition du substrat 3-hydroxy-alcanoate ou de l'ATP.

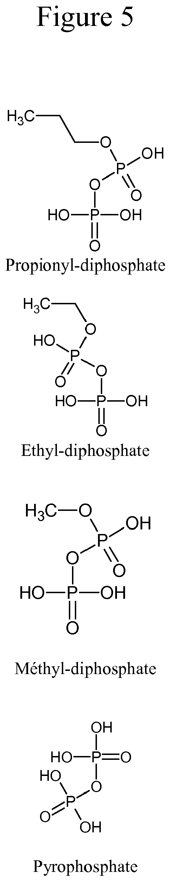

Dans un mode de réalisation préféré, des co-facteurs sont ajoutés de façon à mimer au mieux la réaction naturelle. En effet, les 3-hydroxy-alcanoates ont une structure correspondant généralement à un fragment du MDP, et un espace important de la gorge catalytique est laissé vide lors de l'association enzyme/substrat. Le comblement de cet espace par un co-facteur remplaçant la partie manquante dans le substrat a pour fondement de miner au plus proche la molécule de MDP. Ne se
trouvant pas modifié par la réaction, le co-facteur devra donc n'être ajouté à la réaction qu'en quantités catalytiques. Dans le cas où le substrat de la réaction est le 3-hydroxy-propionate, le co-facteur complémentaire sera le propyl-diphosphate. Dans le cas où le substrat est le 3-hydroxy-butyrate ou le 3-hydroxy-3-méthyl-butyrate, le co-facteur complémentaire sera l'éthyl-diphosphate. Dans le cas où le substrat est le 3-hydroxy-valérate ou le 3-hydroxy-3-méthyl-valérate, le co-facteur complémentaire sera le méthyl-diphosphate. Ces différentes molécules sont présentées en figure 5. Incidemment, il pourra survenir que le co-facteur complémentaire d'une réaction ait un effet positif sur la réaction d'un autre substrat. Généralement, le co-facteur peut être n'importe quelle molécule comprenant un phosphoanhydride, et ayant donc pour formule globale R-PO₂H-O-PO₃H₂, dans laquelle R est notamment H, un résidu alcoyle, linéaire, branché ou cyclique, ou tout autre radical organique monovalent. Les motifs analogues correspondant aux mono-esters du methylène diphosphonate, de formule générale R-O-PO₂H-CH₂-PO₃H₂, dans lesquels le phosphoanhydride est remplacé par un pont méthylène présentant l'avantage de ne pas s'hydrolyser, peuvent également constituer le co-facteur. Plus généralement, les co-facteurs peuvent être les analogues monophosphates, voire sans phosphate, des molécules précédentes, ou encore toute autre molécule susceptible d'améliorer le rendement de la réaction en effectuant une complémentation stérique ou électronique dans le site catalytique de l'enzyme. Dans un mode de réalisation particulier, un co-substrat est ajouté à la réaction. Ledit co-substrat peut être soit l'ATP, c'est-à-dire le co-substrat naturel de la MDP décarboxylase, soit n'importe quel rNTP (ribonucléoside triphosphate) ou dNTP (désoxyribonucléoside triphosphate) ou n'importe quel mélange de rNTP ou dNTP, ou encore du pyrophosphate, ou un autre polyphosphate.

Dans un mode préféré de réalisation, on utilise pour transformer un 3-hydroxy-alcanoate comme défini ci-dessus en alcène terminal une enyzme présentant un homologie de séquence d'au moins 15%, et de préférence d'au moins 30%, 50%, et encore plus préférentiellement d'au moins 80, 90, 95, 96, 97, 98 ou 99% avec une des enzymes correspondant aux séquences SEQ ID NO:1 à 16. Notamment, l'enzyme peut avoir été modifiée par ingénierie à partir de l'une des enzymes SEQ ID NO:1 à 16, ou de toute autre mevalonate diphosphate décarboxylase identifiée à partir d'autres origines. Une telle enzyme peut avoir perdu son activité MDP décarboxylase notamment par ingéniere du gène en laboratoire, mais aussi lors de l'évolution naturelle (on peut alors parler de vestige de MDP décarboxylase) et conservé ou accru son activité sur une ou plusieurs molécules de type 3-hydroxy-alcanoate comme défini ci-dessus. La génération de variants des ces enzymes, plus réactifs envers lesdits substrats, permet d'améliorer le rendement de la réaction selon l'invention. Ainsi, la réactivité de la MDP décarboxylase de type sauvage vis-à-vis des 3-hydroxy-alcanoates comme défini ci-dessus n'est pas nécessairement optimale. Toutes les approches connues de l'homme du métier pour réaliser et sélectionner de tels variants, telles que la mutagenèse aléatoire, la mutagenèse dirigée, la mutagenèse massive, le mélange de gènes (DNA-shuffling), ou l'évolution *in vivo* peuvent être utilisées. Il est également décrit l'utilisation d'une enzyme totalement artificielle, obtenue par conception et réalisation d'un gène synthétique spécifiant une enzyme inédite dans le but de convertir un 3-hydroxy-alcanoate comme défini ci-dessus en alcène terminal, en utilisant ou non les données connues des MDP décarboxylases pour le concevoir.

Un autre objet de l'invention concerne l'utilisation d'une enzyme ayant une activité décarboxylase et comprenant tout ou partie de la séquence SEQ ID NO : 6, ou d'une enzyme présentant une homologie de séquence telle que décrite ci-dessus, pour produire un alcène terminal. Dans une variante, la séquence peut comprendre en outre des résidus supplémentaires, comme par exemple une étiquette Histidineen N-terminal. Il est aussi décrit i un procédé de production d'une enzyme ayant une activité décarboxylase et comprenant tout ou partie de la séquence SEQ ID NO : 6, ou d'une enzyme présentant une homologie de séquence telle que décrite ci-dessus, le procédé comprenant la culture d'un microorganisme comprenant un acide nucléique recombinant codant ladite séquence dans des conditions permettant l'expression de cette séquence. Dans ce contexte, il est décrit, en plus de l'acide nucléique natif (SEQ ID NO : 19), un acide nucléique présentant une séquence optimisée pour l'expression de l'enzyme SEQ ID NO : 6 dans des bactéries, notamment dans E. *coli* (SEQ ID NO: 17). Cet acide nucléique, de même que tout acide nucléique optimisé (c'est-à-dirse permettant une expression améliorée de 30% au moins par rapport à la séquence sauvage) peut être utilisé dans la présente demande. La présente demande décrit aussi un microorganisme comprenant un acide nucléique recombinant codant une enzyme ayant une activité décarboxylase et comprenant tout ou partie de la séquence SEQ ID NO : 6, ou d'une enzyme présentant une homologie de séquence telle que décrite ci-dessus. Le microorganisme est de préférence une bactérie, une levure ou un champignon. La présente demande décrit également toute plante ou animal non humain comprenant un acide nucléique recombinant codant une mévalonate diphosphate décarboxylase.

Dans un mode de réalisation, la MDP décarboxylase est utilisée sous une forme purifiée pour transformer des 3-hydroxy-alcanoates comme définis ci-dessus en alcènes terminaux. Toutefois, les coûts associés à ce procédé sont élevés, les coûts de production et de purification de l'enzyme et des substrats étant importants.

Dans un autre mode de réalisation, la MDP décarboxylase est présente dans la réaction sous forme d'un extrait non purifié, ou encore sous forme de bactéries non lysées, afin d'économiser les coûts de purification de la proténe. Toutefois, les coûts associés à ce procédé sont encore assez élevés à cause des coûts de production et de purification des substrats.

Dans un autre mode de réalisation de l'invention, le procédé met en oeuvre un microorganisme vivant ou une plante vivante produisant l'enzyme permettant de réaliser la conversion. L'invention est ainsi également caractérisée par la modification par génie génétique d'une souche bactérienne productrice d'un ou de plusieurs 3-hydroxy-alcanoates comme définis ci-dessus par exemple *Alcaligenes eutrophus* ou *Bacillus megaterium,* ou encore une souche de *E. coli* modifiée en laboratoire de façon à produire le ou lesdits produits), de façon à ce que ladite souche bactérienne surexprime la décarboxylase, ladite enzyme étant de préférence issue d'un organisme différent du microorganisme hôte, et puisse générer directement un ou plusieurs alcènes terminaux. La modification génétique peut consister en une intégration chromosomique d'un gène de la décarboxylase, l'expression de l'enzyme par un plasmide contenant un promoteur en amont de la séquence codante de l'enzyme, le promoteur et la séquence codante étant de préférence issus d'organismes différents, ou de tout autre moyen connu de l'homme du métier. Alternativement, d'autres bactéries ou levures peuvent présenter des avantages particuliers et être retenus. Ainsi, une levure telle que *Saccharomyces cerevisiae,* une bactérie extrémophile telle que *Thermus thermophilus,* ou des bactéries anaérobies de la famille des *Clostridiae* par exemple, des microalgues, des bactéries photosynthétiques peuvent être utilisées. De façon à produire optimalement le ou les 3-hydroxy-alcanoate, qui seront ensuite convertis en alcènes terminaux, les souches peuvent également avoir été modifiées par génie génétique, c'est-à-dire par recombinaison *in vitro* ou par évolution dirigée *in vivo.*

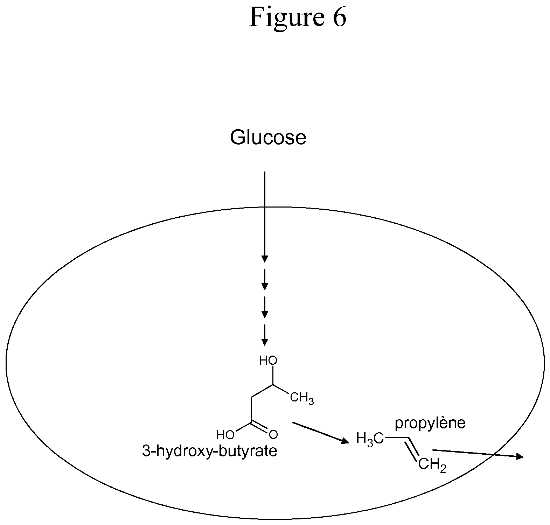

Selon un mode de réalisation possible, un procédé de l'invention est caractérisé par la transformation d'une
source de carbone telle que le glucose, en 3-hydroxy-alcanoate, puis par la transformation dudit produit primaire en produit secondaire, c'est-à-dire en alcène terminal. Les différentes étapes dudit procédé sont explicitées en figure 6.

Dans un mode de réalisation particulier, l'invention est caractérisée par la transformation de polyhydroxyalcanoates en 3-hydroxy-alcanoate comme défini ci-dessus, en utilisant une enzyme ou un procédé physico-chimique adapté, puis par la transformation dudit produit primaire en produit secondaire, c'est-à-dire en alcène terminal. Eventuellement, le polyhydroxyalcanoate a été produit par une plante ayant été modifiée au niveau de ses voies métaboliques de façon à produire de hauts rendements de polyhydroxyalcanoate.

Dans un mode de réalisation particulier, l'invention consiste en le procédé intégré de production des produits à partir de CO₂ atmosphérique ou artificiellement ajouté au milieu de culture. Le procédé de l'invention est réalisé dans un organisme capable d'effectuer de la photosynthèse, tel que les microalgues par exemple.

Dans ces modes de réalisation, le procédé de l'invention est encore caractérisé par le mode de récupération des produits, qui dégazent de la culture. Les alcènes terminaux courts, et notamment l'éthylène, le propylène, les isomères du butène, prennent en effet un état gazeux à température ambiante et à la pression atmosphérique. Le procédé de l'invention ne requiert donc pas d'extraction du produit à partir du milieu liquide de la culture, étape qui constitue toujours une source de coûts importante au niveau industriel. L'évacuation et le stockage des hydrocarbures gazeux, et leur éventuelle séparation physique et conversion chimique ultérieures, peuvent être opérés suivant tout procédé connu de l'homme du métier.

Dans un mode de réalisation particulier, l'invention comprend également la détection de l'alcène (propylène, d'éthylène et d'iso-butylène notamment) présent dans la phase gazeuse du procédé. La présence des composés ciblés dans un environnement d'air ou d'un autre gaz, même en faibles quantités, peut se faire en utilisant diverses technologies, et notamment en utilisant des systèmes de chromatographie en phase gazeuse et la détection aux infrarouges, par ionisation de flamme, ou par couplage avec un spectromètre de masse.

Dans un mode de réalisation particulier, les alcènes terminaux obtenus sont condensés, puis éventuellement réduits, en utilisant des techniques connues de l'homme du métier, afin de produire des alcènes de plus longue chaîne, ou des alcanes de plus longue chaîne. En particulier, l'iso-butylène peut être utilisé pour synthétiser de l'iso-octane : les procédés catalytiques pour mener à bien cette réaction sont déjà amplement décrits.

Dans un mode de réalisation particulier, le procédé implique la culture des micro-organismes dans des conditions de culture habituelles (30 à 37°C sous 1 atmosphère, dans un fermenteur permettant la croissance aérobie des bactéries) ou non habituelles (température plus élevée pour correspondre aux conditions de culture d'un organisme thermophile, par exemple).

Dans un mode de réalisation particulier, les micro-organismes sont cultivés en microaérophilie, la quantité d'air injectée étant limitante afin de minimiser la concentration de dioxygène résiduelle dans les effluents gazeux chargés d'hydrocarbures alcéniques.

D'autres aspects et avantages de l'invention seront décrits dans les exemples qui suivent, qui doivent être considérés comme illustratifs et non limitatifs.

### Légende des Figures

Figure 1 : Motif 3-hydroxy-propionate
Figure 2: Décarboxylation du mévalonate di-phosphate par la MDP-décarboxylase - activité générique
Figure 3 : Exemples de 3-hydroxy-alcanoates.
Figure 4 : Utilisation de la MDP décarboxylase pour générer des alcènes terminaux
Figure 5 : Co-facteurs utilisables pour la réaction afin de réaliser la complémentation structurelle dans le site catalytique
Figure 6 : Procédé intégré de production d'un alcène à partir de glucose
Figure 7 : Chromatogramme réalisé sur des réactions enzymatiques menées dans les conditions n°1 de l'exemple 4.
Figure 8 : SDS-PAGE des étapes de surexpression et purification de l'enzyme SEQ ID NO 6.
   1. Marqueurs
   2. Culture avant induction
   3. Lysat
   4. Fraction non absorbée sur la colonne
   5. Fraction de lavage de la colonne
   6. Enzyme purifiée, Mw=36,8 kDa
Figure 9 : Chromatogramme d'une analyse de la réaction de conversion de *HIV* en *IBN* par CPG/MS. 1 et 2 : Contrôles négatifs correspondants au bruit de fond observé sans enzyme 3 et 4 : Réactions en présence de l'enzyme SEQ ID NO : 6.
Figure 10 : Ratio de la production d'IBN en présence et absence d'ATP.
Figure 11 : Ratio de la production d'IBN en présence et absence de Mg²⁺.
Figure 12 : Activité enzymatique en fonction de la température. Ratio : quantité d'IBN formée en présence d'enzyme par rapport au bruit de fond.
Figure 13 : Production de l'IBN en fonction de la concentration du substrat HIV.
Figure 14 : Mesure de la réaction optimisée et comparaison au bruit de fond. Mesure par chromatographie gazeuse couplée à un détecteur à ionisation de flamme.
Figure 15 : Amélioration du niveau d'expression par optimisation de la séquence nucléotidique codant SEQ ID NO : 6. Piste M : marqueurs de poids moléculaire
   Pistes 1, 2, 3: séquence nucléotidique native
      (1) Lysat cellulaire, fraction soluble déposée sur la colonne de purification
      (2) Fraction de lysat non retenue par la colonne de purification
      (3) Fraction éluée: 10µg enzyme purifiée
   Pistes 4, 5, 6: Séquence nucléotidique optimisée
      (4) Lysat cellulaire, fraction soluble déposée sur la colonne de purification
      (5) Fraction de lysat non retenue par la colonne de purification
      (6) Fraction éluée: 10µg enzyme purifiée

### Exemples

### Exemple 1 : clonage et expression de plusieurs MDP décarboxylases.

Le gène codant pour la MDP décarboxylase de *Saccharomyces cerevisiae* est généré par synthèse à partir d'oligonucléotides chevauchants, puis inséré dans un plasmide pET (Novagen) permettant l'expression en bactéries. Ledit plasmide est ensuite transformé par électroporation dans des bactéries de souche BL21 (Invitrogen). Les bactéries sont étalées sur une boite de Pétri contenant de l'ampicilline, et mises à incuber à 37°C. Le jour suivant, une colonie bactérienne est sélectionnée au hasard, et utilisée pour ensemencer une culture de 50 ml de milieu LB contenant de l'ampicilline. La culture est incubée pendant 24 heures sous agitation. A l'issue des 24 heures, la culture est centrifugée, les bactéries sont lysées par sonication, et un extrait protéique total est réalisé. Un aliquote de l'extrait est déposé sur un gel l'électrophorèse, en même temps qu'un extrait protéique provenant de bactéries de la même souche, mais non transformées, et d'un marqueur de poids moléculaire. On observe sur la piste correspondant à la souche bactérienne transformée une bande unique à 30kD environ, correspondant à la taille attendue pour la protéine, cette bande n'existant pas sur la piste correspondant aux bactéries non transformées.

### Exemple 2 : mesure de l'activité des extraits protéiques pour le 3-hydroxy-3-méthyl-butyrate.

Du 3-hydroxy-3-méthyl-butyrate (commandé chez Sigma, référence 55453 sous le nom de β-hydroxyisovaleric acid), et suspendu à la concentration de 10 g/L. Du mévalonate-diphosphate est synthétisé à partir de mevalonolactone et d'autres réactifs (Sigma), selon la technique classique, et est resuspendu à la concentration de 10 g/L.

On prépare six flacons de chromatographie. Dans tous les flacons, on introduit 50 µL de tampon contenant 50 mM de Bistris/HCl, 1 mM de dithiothréitol, 10 mM de MgCl2, 5 mM d'ATP.

Dans les flacons 1 et 4, on n'ajoute 5 µL d'eau (aucun substrat).

Dans les flacons 2 et 5, on ajoute, 5 µL de la préparation de mévalonate di-phosphate (contrôle positif)

Dans les flacons 3 et 6, on ajoute 5 µL de la préparation de 3-hydroxy-3-méthyl-butyrate (HIV)

Dans les flacons 1, 2, et 3, on ajoute ensuite 5 µL d'eau (aucune enzyme)

Dans les flacons 4,5, et 6, on ajoute 5 µL de la préparation enzymatique décrite dans l'exemple 1.

On scelle les flacons en utilisant un bouchon à septum et une pince à sceller. On incube tous les flacons à 37°C pendant 4 heures à 3 jours.

A l'issue de cette incubation, on prélève un échantillon du gaz présent dans chaque flacon en utilisant une seringue à gaz, et on mesure la concentration du CO₂ dans ces échantillons en utilisant la chromatographie en phase gazeuse. On s'aperçoit que la concentration en CO₂ est très significative dans le flacon 5, et qu'une concentration en CO₂, plus faible, est également observable dans le flacon 6, ce qui reflète une réaction significative de la préparation enzymatique sur le 3-hydroxy-3-méthyl-butyrate.

On mesure ensuite la présence d'iso-butylène dans l'échantillon de gaz correspondant au flacon 6 en utilisant un système de chromatographie gazeuse couplée à un détecteur à infra-rouges ou à ionisation de flamme.

### Exemple 3 : optimisation des conditions de réaction en utilisant un co-facteur.

On effectue la même réaction que celle décrite dans le flacon 6 de l'exemple précédent, mais en utilisant, dans l'un des échantillons, de l'éthyl-diphosphate comme co-facteur, synthétisé à façon.

Dans cet exemple, on dispose de trois flacons. Le premier contient les tampons, l'ATP, l'extrait enzymatique dans les quantités décrites dans l'exemple précédent. Le second contient les même éléments, avec en plus du 3-hydroxy-3-méthyl-butyrate dans les quantités décrites dans l'exemple précédent. Le troisième flacon contient, en plus du 3-hydroxy-3-méthyl-butyrate, 10µL d'éthyl-diphosphate à 10mg/L.

On mesure, comme dans l'exemple précédent, la formation de l'iso-butylène en utilisant un appareillage de chromatographie gazeuse couplée à de la détection aux infra-rouges ou à ionisation de flamme. On s'aperçoit que lorsque l'éthyl-diphosphate est présent, la quantité d'iso-butylène produite par unité de temps est nettement supérieure.

### Exemple 4. Criblage d'une collection d'enzymes

Une collection de soixante trois gènes codant pour des enzymes de la famille de la MDP décarboxylase ont été obtenus et testés pour leur activité sur le substrat HIV.

*Clonage, cultures bactériennes et expression des protéines.*
Les gènes correspondant à la famille diphosphate mevalonate (MDP) décarboxylases EC 4.1.1.33 ont été clonés dans le vecteur pET 25b (Novagen) pour les gènes d'origine eucaryote et pET 22b (Novagen) pour les gènes d'origine procaryote avec 6 résidus His ajoutés en N-terminal, juste après le codon méthionine initiateur. Les cellules compétentes *E.coli* BL21(DE3) (Novagen) ont été transformées avec les vecteurs par choc thermique. Les cellules ont été cultivées sur le milieu TB contenant 0,5 M sorbitol, 5 mM betaïne, 100 µg/ml ampicilline à 30°C, sous agitation (160 rpm) jusqu'à une DO (600 nm) comprise entre 0,8 et 1. L'isopropyl-B-D-thiogalactopyranoside (IPTG) a ensuite été ajouté à la concentration finale 1 mM et l'expression des protéines a été poursuivie à 20°C durant la nuit (environ 16H). Les cellules ont été collectées par centrifugation à 4°C, 10 000 rpm, 20 minutes et les culots congelés à -80°C.

### Lyse des cellules

1,6 g de cellules a été décongelé sur glace ; les cellules ont été re-suspendues dans 5 ml de 50 mM Na2HPO4 contenant 300 mM NaCl, 5 mM MgCl2, 1 mM DTT pH 8. 20 µl de lysonase (Novagen) ont été rajoutés puis une incubation 10 min à la température ambiante a été menée, et suivie d'un retour 20 min sur glace. La lyse des cellules bactériennes a été complétée par sonication dans un bain-marie à ultrason 3 fois 5 minutes à 0°C avec une homogénéisation des échantillons entre les pulses. Les extraits bactériens ont été ensuite clarifiés par centrifugation à 4°C, 10 000 rpm, 20 minutes.

### Purification et concentration des protéines (kit PROTINO)

Des lysats bactériens clarifiés ont été déposés sur la colonne PROTINO-1000 Ni-IDA (Macherey-Nagel) permettant l'absorption des protéines portant une étiquette de 6 histidines consécutives (His tag). Les colonnes ont été lavées et les enzymes d'intérêt ont été éluées avec 4 ml de 50 mM Na2HPO4 contenant 300 mM NaCl, 5 mM MgCl2, 1 mM DTT, 250 mM imidazole pH 8. Les éluats ont ensuite été concentrés dans des cellules Amicon Ultra-4 10 kDa (Millipore) jusqu'à un volume final de 250 µL. La quantité d'enzymes a été déterminée par la méthode Bradford.

### Réactions enzymatiques

L'activité enzymatique recherchée (conversion du 3-méthyl 3-hydroxy butyrate, ou 3-hydroxy isovalerate, ou encore HIV) a été testée dans deux conditions expérimentales différant par le tampon et le pH de la réaction.

Conditions expérimentales N°1.
100 mM citrate
10mM MgCl2
10 mM ATP
20 mM KCl
200 mM HIV
pH final a été ajusté à 5,5

Conditions expérimentales N°2.
100 mM Tris-HCl pH 7,0
10mM MgCl2
10 mM ATP
20 mM KCl
200 mM HIV
pH final a été ajusté à 7,0

L'enzyme a ensuite été ajoutée au mélange réactionnel. Le rendement de production de protéine étant variable, la quantité d'enzyme ajoutée variait d'un échantillon à l'autre entre 0,01 et 1 mg/ml. Les réactions de contrôle, sans enzyme, ont été menées en parallèle.

Les réactions (1 mL) on été placées dans des flacons de 2 ml (Interchim), scellés avec des septums en téflon/silice/téflon (Interchim). Les réactions ont été incubées à 37 °C sans agitation pendant 72 heures.

### Analyse des réactions

Le gaz présent au-dessus des réactions a été prélevé avec une seringue équipée d'un système anti-retour. Le gaz a ensuite été analysé par chromatographie en phase gazeuse (CPG) couplé à un spectromètre de masse (SM). L'appareil était préalablement étalonné en utilisant une gamme de concentration d'isobutylène.
Colonne : BPX5 (SGE)
CPG/SM : MSD 5973 (HP)

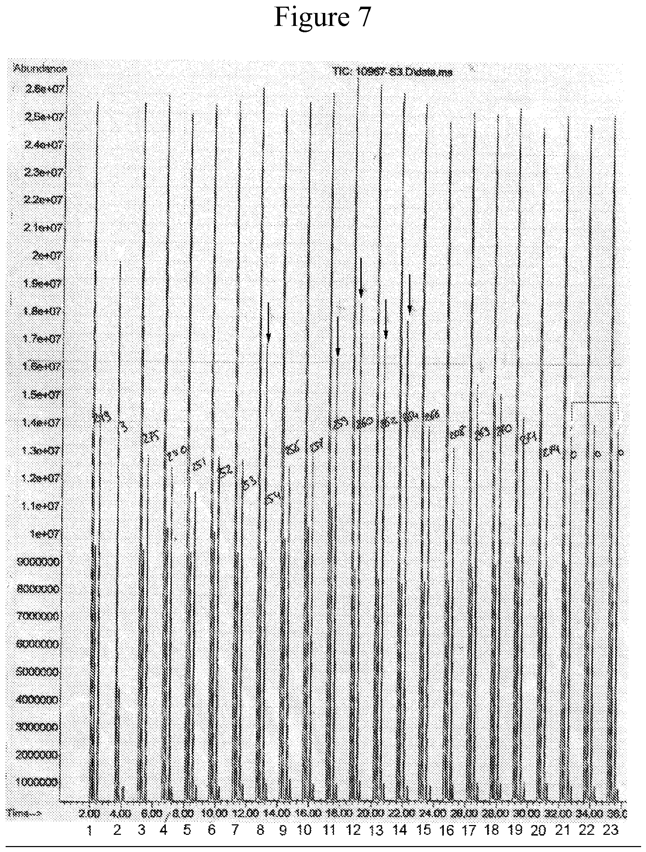

Pour chaque chromatogramme réalisé, on obtient trois pics principaux, le premier correspondant à l'air, le second à l'eau, et le troisième à l'isobutylène. Sur les soixante trois enzymes produites et testées, onze candidats potentiels ont été identifiés au premier criblage. Certains de ces candidats sont identifiés par une flèche sur la Figure 7. Leur identité est présentée dans la liste ci-dessous, et leurs séquences sont présentées dans les SEQ NO 6 à 16 (his-tag non figuré).
**Candidat 1 : SEQ ID NO: 7**
   Numéro d'accession Genebank: CAI97800.1
   Numéro d'accession Swissprot/TrEMBL : Q1 GAB2
   Microorganismes: Lactobacillus delbrueckii subsp. bulgaricus (souche ATCC 11842 / DSM 20081)
**Candidat 2: SEQ ID NO: 8**
   Numéro d'accession Genebank : CAJ51653
   Numéro d'accession Swissprot/TrEMBL : Q18K00
   Microorganismes: Haloquadratum walsbyi DSM 16790
**Candidat 3: SEQ ID NO: 9**
   Numéro d'accession Genebank : ABD99494.1
   Numéro d'accession Swissprot/TrEMBL : Q1WU41
   Microorganismes: Lactobacillus salivarius subsp. salivarius (strain UCC118)
**Candidat 4: SEQ ID NO: 10**
   Numéro d'accession Genebank : ABJ57000.1
   Numéro d'accession Swissprot/TrEMBL : Q04EX2
   Microorganismes: Oenococcus oeni (strain BAA-331 / PSU-1)
**Candidat 5: SEQ ID NO: 11**
   Numéro d'accession Genebank : ABJ67984.1
   Numéro d'accession Swissprot/TrEMBL : Q03FN8
   Microorganismes: Pediococcus pentosaceus ATCC 25745
**Candidat 6: SEQ ID NO: 12**
   Numéro d'accession Genebank : ABV09606.1
   Numéro d'accession Swissprot/TrEMBL : A8AUU9
   Microorganismes: Streptococcus gordonii (souche Challis / ATCC 35105 / CH1 / DL1 / V288)
**Candidat 7: SEQ ID NO: 13**
   Numéro d'accession Genebank : ABQ14154.1
   Numéro d'accession Swissprot/TrEMBL : A5EVP2
   Microorganismes: Dichelobacter nodosus VCS1703A
**Candidat 8: SEQ ID NO: 14**
   Numéro d'accession Genebank : EDT95457.1
   Numéro d'accession Swissprot/TrEMBL : B2DRT0
   Microorganismes: Streptococcus pneumoniae CDC0288-04
**Candidat 9: SEQ ID NO: 15**
   Numéro d'accession Genebank : AAT86835
   Numéro d'accession Swissprot/TrEMBL : Q5XCM8
   Microorganismes: Streptococcus pyogenes sérotype M6 (ATCC BAA-946 / MGAS10394)
**Candidat 10 : SEQ ID NO: 6**
   Numéro d'accession Genebank : AAT43941
   Numéro d'accession Swissprot/TrEMBL : Q6KZB1
   Microorganismes: Picrophilus torridus DSM 9790
**Candidat 11 : SEQ ID NO: 16**
   Numéro d'accession Genebank : AAV43007.1
   Numéro d'accession Swissprot/TrEMBL : Q5FJW7
   Microorganismes: Lactobacillus acidophilus NCFM

La production la plus élevée d'IBN a été observée avec le candidat 10, c'est-à-dire avec l'enzyme décarboxylase purifiée de SEQ ID NO : 6 provenant de *Picrophilus torridus.* Cette enzyme a été retenue pour être caractérisée en détail.

### Exemple 5 : Caractérisation de l'enzyme de SEQ ID NO : 6

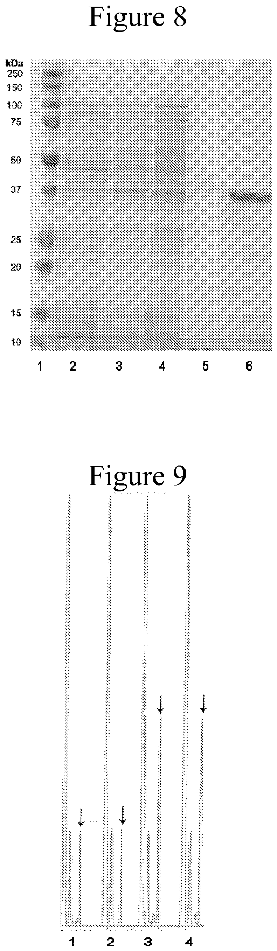

L'enzyme recombinante a été purifiée comme décrit dans l'exemple 4. Les résultats, présentés sur la Figure 8, montrent que la pureté de l'enzyme dans l'échantillon protéique final est estimée à 90%.

L'activité de l'enzyme isolée a été confirmée. La réaction a été menée dans les conditions suivantes :
100 mM Tris-HCl pH 7,0
10mM MgCl2
10 mM ATP
20 mM KCl
250 mM HIV
pH final a été ajusté à 6,0
3 mg/mL enzyme

Après une incubation à 30°C pendant 72h, on mesure le signal par CPG/SM. Les résultats sont présentés sur la Figure 9. En présence d'enzyme, la production de l'IBN est augmentée ici d'un facteur environ 2,3 en comparaison du bruit de fond. Le bruit de fond observé ici est homogène avec la littérature de la chimie organique, qui mentionne que l'acide 3-hydroxy-isovalérique en solution aqueuse et à une température de l'ordre de 100°C se décarboxyle lentement en tertio-butanol, lequel se convertit partiellement par déshydratation en isobutylène, suivant un équilibre favorable à la formation de tertio-butanol (Pressman et Luca, J. Am. Chem. Soc. 1940).

### Effet du co-substrat ATP

Conditions de test
100 mM citrate
50 mM KCI
10 mM MgCl2
200 mM HIV (à préciser)
1 mg/ml enzyme purifiée
pH 5,5
Incubation à 30°C pendant 72 H

| Conditions | Concentration finale ATP | Enzyme |
|---|---|---|
| 1 | 0 mM | 0 mg/mL |
| 2 | 0 mM | 1 mg/mL |
| 3 | 10 mM | 0 mg/mL |
| 4 | 10 mM | 1 mg/mL |

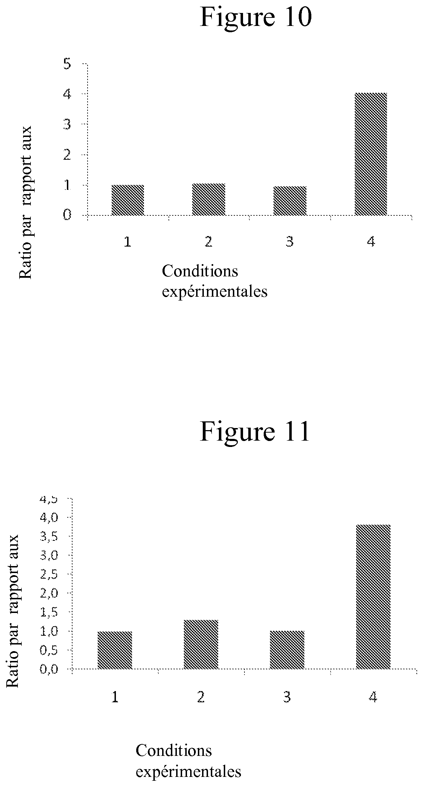

Les résultats sont présentés sur la figure 10 et montrent que l'activité enzymatique n'est observée qu'en présence de co-substrat ATP. D'autres molécules, et notamment des molécules contenant une liaison phospho-anhydride, sont susceptibles de constituer des co-substrats efficaces pour l'enzyme.

### Effet du co-facteur Mg²⁺

### Conditions de test

100 mM citrate pH 5,5
50 mM KCI
10 mM ATP
200 mM HIV (à préciser)
pH 5,5
1 mg/ml enzyme purifiée
Incubation à 30°C pendant 72 H

| Conditions | Concentration finale MgCl2 | Enzyme |
|---|---|---|
| 1 | 0 mM | 0 mg/mL |
| 2 | 0 mM | 1 mg/mL |
| 3 | 5 mM | 0 mg/mL |
| 4 | 5 mM | 1 mg/mL |

Les résultats sont présentés sur la figure 11 et montrent que l'activité de l'enzyme est améliorée en présence d'ions Mg ²⁺. D'autres ions, et notamment d'autres ions divalents, sont susceptibles d'être utilisés comme co-facteur en substitution ou en plus des ions Mg²⁺.

### Activité enzymatique en fonction de la température

### Conditions des tests

100 mM tampon
50 mM KCI
10 mM ATP
200 mM HIV (à préciser)
1 mg/ml enzyme purifiée
Incubation pendant 72 H à une température variable.

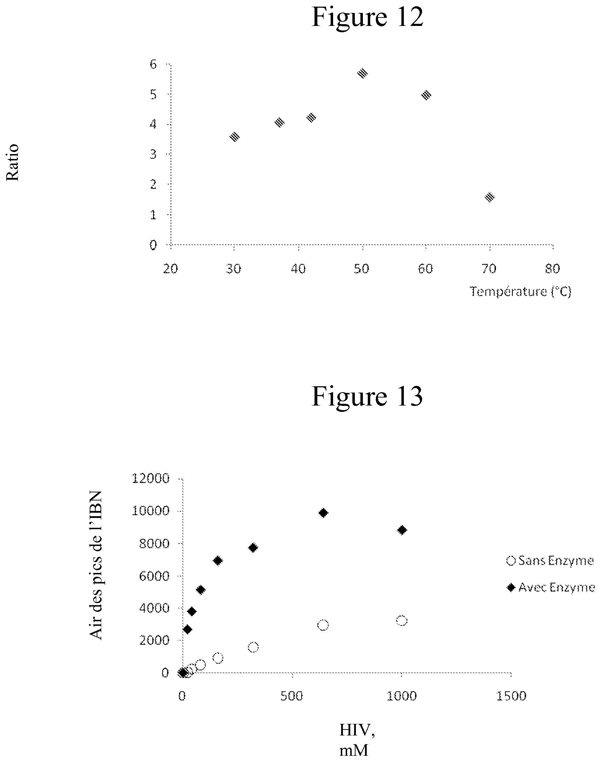

Les résultats présentés sur la figure 12 montrent que l'enzyme est modérément thermoactive et présente un optimum de température d'environ 50°C.

### Activité en fonction de pH

### Conditions des tests

100 mM tampon
50 mM KCl
10 mM ATP
200 mM HIV (à préciser)
1 mg/ml enzyme purifiée
Incubation à 30°C pendant 72 H

Les conditions optimales a été obtenues à un pH de 5,5, dans 100 mM citrate.

### Paramètres enzymatiques

Une gamme de substrat a été réalisée dans les conditions précédemment décrites, avec une incubation à 50°C. Le Km de l'enzyme est estimé à 40 mM d'HIV.

### Optimisation des conditions réactionnelles

Les conditions réactionnelles optimales ont été recherchées. Les conditions suivantes ont été retenues :
100 mM citrate
50 mM KCI
40 mM ATP
200 mM HIV
1 mg/ml enzyme
Incubation à 50°C pendant 48 H

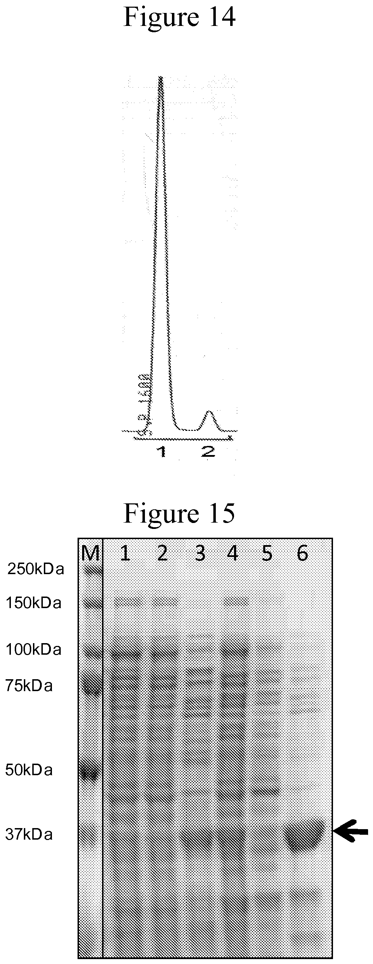

Comme montré sur la figure 14, le signal rapport sur bruit de fond est d'environ 100.

### Exemple 6. Optimisation du niveau d'expression de la MDP décarboxylase de P. torridus dans E. coli

Le niveau d'expression initial dans E. *coli* BL21 était faible, puisque la bande était difficilement visible en SDS-PAGE avant purification. Le Codon Optimisation Index (CAI) de la séquence native pour une expression en E. *coli* a été mesuré en utilisant le logiciel « Optimizer » disponible sur http://genomes.urv.es/OPTIMIZER/, et s'inspirant de la méthode de Sharp et Li (1987). La valeur obtenue était de seulement 0,23, reflétant le niveau d'expression limité de la protéine étudiée dans E *coli.*

Une séquence codant pour une protéine identique, mais ayant des codons mieux adaptés à l'expression dans *E*. *coli* a été générée. Le CAI de cette séquence, 0,77 était plus proche de l'optimum de 1.

La séquence native et la séquence optimisée sont présentées SEQ ID NO : 17 (Séquence optimisée de la MDP décarboxylase de P. torridus (AAT43941) incluant le His Tag) et SEQ ID NO : 19 (Séquence native de la MDP décarboxylase de P. torridus (AAT43941) incluant le His Tag).

La séquence optimisée a été synthétisée par concaténation d'oligonucléotides, et clonée dans un vecteur d'expression pET25. Après transformation du vecteur dans la souche *E.coli* BL21(DE3) et induction en utilisant le protocole précédemment décrit, les protéines ont été produites, purifiées et analysées sur gel de façon similaire à celle décrite précédemment. Le même protocole a été mené avec la séquence native à titre de comparaison.

Comparaison des niveaux d'expression du candidat 224 en utilisant soit la séquence nucléotidique native soit la séquence optimisée pour l'expression dans *E.coli.*

Les résultats présentés en Figure 15 montrent que la protéine correspondant au gène optimisé est clairement visible sur gel dans le lysat cellulaire non purifié (piste 4), ce qui traduit un gain d'expression très significatif. La pureté de la protéine après l'étape de purification est également plus importante dans le cas du gène optimisé.

L'activité a été mesurée sur le lysat brut. En effet, cette activité était indétectable sur le lysat brut obtenu avec la séquence nucléique native. L'expression de la protéine a été améliorée de telle manière que le lysat brut obtenu à partir de la séquence nucléique améliorée (clone 224 optimisé) présente maintenant cette activité.

Le milieu réactionnel utilisé dans ce test a été le suivant :

**Milieu réactionnel**

| Produits | Concentration finale |
|---|---|
| Tampon de réaction Acetate Buffer (500 mM, pH=5,5) | 50 mM |
| MgCl2 (1M) | 10 mM |
| KcL (1 M) | 20 mM |
| HIV (3M) | 50 mM |
| ATP (100 mM) | 40 mM |
| Inhibiteur de protéases (100X) | 1X |
| H20 | |
| Enzyme | 89 ug de protéines totales (lysat brut) |

Incubation 2 jours à 50°C.

### Résultats

Condition n°1 : Lysat du clone 224 optimisé
Condition n°2 : Lysat du clone GB6 (plasmide pET vide)

| Conditions | Aire du signal | Ratio |
|---|---|---|
| 1 | 1083 | 22 |
| 2 | 49 | |

### Exemple 7 : procédé de synthèse d'iso-butylène à partir de 3-hydroxy-3-méthyl-butyrate et conversion en iso-octane.

Une réaction identique à celle du flacon 3 de l'exemple 3 est réalisée dans un volume de 1 litre, disposé dans un fermenteur disposant d'un système d'extraction gazeuse. La présence de l'enzyme recombinante induit la conversion du 3-hydroxy-3-méthyl-butyrate en iso-butylène, qui dégaze naturellement, et est récupéré par un système d'extraction gazeuse situé dans la partie supérieure du fermenteur. L'iso-butylène est ensuite utilisé pour générer de l'iso-octène par addition catalysée par la résine Amberlyst 35wet ou 36wet (Rohm and Haas). L'iso-octène est enfin réduit par hydrogénation catalytique en iso-octane.

### Exemple 8 : ingénierie de l'enzyme pour améliorer son efficacité pour les substrats.

On utilise la technologie de mutagenèse aléatoire pour générer une banque contenant des milliers de mutants à partir du gène décrit dans l'exemple 1. On intègre ensuite cette banque de mutants dans le plasmide d'expression, et on transforme la banque dans des bactéries compétentes de souche BL21. On isole ensuite un millier de bactéries, qu'on repique dans des tubes eppendorf contenant 500µL de milieu LB supplémenté en ampicilline. On incube ces échantillons dans un agitateur pendant 15 heures. Le lendemain, on mesure la quantité d'iso-butylène produite en utilisant l'un ou l'autre des protocoles expérimentaux présentés dans les exemples précédents.

Les clones présentant une quantité d'iso-butylène significativement augmentée sont ensuite validés de nouveau en utilisant le même protocole expérimental. Une fois validée leur amélioration, le plasmide est extrait de chaque souche améliorée, et séquencé. Les mutations à l'origine de l'amélioration d'activité sont identifiées, et combinées sur un même plasmide. Le plasmide contenant les différentes mutations améliorantes est à son tour transformé dans des bactéries compétentes, et le même système d'analyse est effectué.

Le clone combinant les différentes mutations, présentant une activité significativement supérieure à celle ne contenant qu'une seule mutation améliorante, est ensuite utilisé comme base pour un nouveau tour de mutation/criblage, à la recherche de mutants présentant une activité encore améliorée.

A l'issue de ce protocole, le clone contenant plusieurs mutations et ayant la meilleure activité est sélectionné.

### Exemple 9 : procédé de synthèse d'éthylène à partir de 3-hydroxy-propionate.

Le gène codant pour l'enzyme décrite dans l'exemple 1 est inséré dans un plasmide permettant l'expression de protéines recombinantes dans une souche d'E. *coli.* Le plasmide est transformé dans des bactéries de ladite souche. Les bactéries transformantes sont ensuite incubées dans un fermenteur en présence de propyl-diphosphate (10 mg/L) et de 3-hydroxy-propionate (1 g/L). La présence de l'enzyme recombinante induit la conversion du 3-hydroxy-propionate en ethylène, qui dégaze spontanément, et est récupéré par un système d'extraction gazeuse situé dans la partie supérieure du fermenteur. On mesure ensuite la présence d'éthylène dans l'échantillon de gaz correspondant en utilisant un système de chromatographie gazeuse couplée à un détecteur à infra-rouges, partie du spectre dans lequel l'éthylène présente une forte émission.

### Exemple 10 : procédé de synthèse de propylène à partir de 3-hydroxy-butyrate.

Le gène codant pour l'enzyme décrite dans l'exemple 1 ou pour une enzyme décrite dans l'exemple 4 est inséré dans un plasmide permettant l'expression de protéines recombinantes dans une souche *d'E. coli.* Le plasmide est transformé dans des bactéries de ladite souche. Les bactéries transformantes sont ensuite incubées dans un fermenteur en présence d'éthyl-diphosphate (10 mg/L) et de 3-hydroxy-butyrate (1 g/L) (Sigma, référence 166898). La présence de l'enzyme recombinante induit la conversion du 3-hydroxy-butyrate en propylène, qui dégaze spontanément, et est récupéré par un système d'extraction gazeuse situé dans la partie supérieure du fermenteur. On mesure ensuite la présence de propylène dans l'échantillon de gaz correspondant en utilisant un système de chromatographie gazeuse couplée à un détecteur à infra-rouges, partie du spectre dans lequel le propylène présente une forte émission.

### Exemple 11 : procédé de synthèse de propylène à partir de glucose.

Le gène codant pour l'enzyme décrite dans l'exemple 1 ou pour une enzyme décrite dans l'exemple 4 est inséré dans un plasmide permettant l'expression de protéines recombinantes dans la bactérie *Alcaligenes eutrophus.* Le plasmide est transformé dans des bactéries de ladite souche.

Les bactéries sont ensuite incubées dans un fermenteur en présence de glucose et d'éthyl-diphosphate et dans des conditions de micro-aérophilie, puis soumise à un stress thermique qui a pour conséquence de leur faire produire de grandes quantités de 3-hydroxy-butyrate. La présence de l'enzyme recombinante induit la conversion simultanée du 3-hydroxy-butyrate en propylène. Le propylène dégaze naturellement, et est récupéré par un système d'extraction gazeuse situé dans la partie supérieure du fermenteur.

### Exemple 12 : procédé de synthèse de propylène à partir de glucose.

Le présent exemple décrit un procédé très voisin de celui de l'exemple 11. La différence principale consiste en l'utilisation d'une souche de E. *coli* modifiée de façon à produire du 3-hydroxy-butyrate et non une souche naturelle telle que *Alcaligenes eutrophus.* Ladite souche été obtenue par ingénierie de voies métaboliques de façon à aboutir à l'accumulation de 3-hydroxy-butyrate. L'ajout d'une MDP décarboxylase telle que décrite dans l'exemple 1 ou dans l'exemple 4 permet de convertir le 3-hydroxy-butyrate en propylène.

### Exemple 13 : procédé de synthèse d'isobutylène à partir de glucose.

Le gène codant pour l'enzyme décrite dans l'exemple 1 est inséré dans un plasmide permettant l'expression de protéines recombinantes dans une souche de bactéries E. *coli* ayant par ailleurs subi des altérations métaboliques de façon à synthétiser de façon endogène du 3-hydroxy-3-méthyl-butyrate.

Les bactéries sont ensuite incubées dans un fermenteur en présence de glucose et dans des conditions de micro-aérophilie. La présence de l'enzyme recombinante induit la conversion simultanée du 3-hydroxy-3-méthyl-butyrate en isobutylène, qui dégaze naturellement et est récupéré par un système d'extraction gazeuse situé dans la partie supérieure du fermenteur.

## Revendications

1. Procédé de production d'un alcène terminal, **caractérisé en ce qu'**il comprend une étape de conversion d'un 3-hydroxy-alcanoate par une enzyme présentant une activité mévalonate diphosphate décarboxylase, dans lequel le 3-hydroxy-alcanoate est un composé de la formule
HO-CO-CH₂-C(R¹)(R²)-OH
ou
O⁻-CO-CH₂-C(R¹)(R²)-OH
dans laquelle R¹ et R² sont choisis, indépendamment, parmi un atome d'hydrogène, un résidu méthyle et un résidu éthyle, et dans lequel la conversion se fait en présence d'un co-substrat, ledit co-substrat étant l'ATP, un rNTP, un dNTP ou un mélange de plusieurs de ces molécules, un polyphosphate, ou le pyrophosphate.

2. Procédé selon la revendication 1, comprenant une étape de conversion de 3-hydroxy-butyrate en propylène.

3. Procédé selon la revendication 1, comprenant une étape de conversion de 3-hydroxy-valérate en 1-butylène.

4. Procédé selon la revendication 1, comprenant une étape de conversion de 3-hydroxy-3-méthyl-butyrate en iso-butylène.

5. Procédé selon la revendication 1, comprenant une étape de conversion de 3-hydroxy-3-méthyl-valérate en iso-amylène.

6. Procédé selon l'une ou plusieurs des revendications précédentes, dans lequel l'enzyme comprend une séquence en acides aminés choisie parmi SEQ ID Nos 1-16, ou une séquence présentant au moins 15% d'homologie de séquence avec l'une de celles-ci.

7. Procédé selon la revendication 6, dans lequel l'enzyme comprend une séquence en acides aminés présentant au moins 50% ou au moins 80% ou au moins 90% d'homologie de séquence avec l'une de séquences SEQ ID NOs: 1-16

8. Procédé selon l'une quelconque des revendications précédentes, **caractérisé en ce que** l'enzyme comprend tout ou partie de la séquence SEQ ID NO : 6, ou d'une séquence présentant au moins 15% d'homologie de séquence avec celle-ci.

9. Procédé selon la revendication 8, dans lequel l'enzyme comprend une séquence en acides aminés présentant au moins 50% ou au moins 80% ou au moins 90% d'homologie de séquence avec SEQ ID NO: 6.

10. Procédé de production d'isobutylène, **caractérisé en ce qu'**il comprend une étape de conversion de 3-hydroxy-3-méthyl-butyrate par une enzyme présentant une activité mévalonate diphosphate décarboxylase.

11. Procédé selon la revendication 10, dans lequel l'enzyme présentant une activité mévalonate diphosphate décarboxylase est une enzyme comprenant tout ou partie de la séquence SEQ ID NO: 6, ou d'une séquence présentant au moins 15% d'homologie de séquence avec celle-ci.

12. Procédé selon la revendication 11, dans lequel l'enzyme comprend une séquence en acides aminés presentant au moins 50% ou au moins 80% ou au moins 90% d'homologie de séquence avec SEQ ID NO: 6.

13. Procédé selon l'une quelconque des revendications précédentes, selon lequel on ajoute à la réaction un co-facteur de formule générale R-O-PO₂H-O-PO₃H₂ dans laquelle R est un atome d'hydrogène, un radical méthyle, éthyle ou propyle, et peut également être tout radical alcoyle, linéaire, branché ou cyclique, ou tout autre radical organique monovalent.

14. Procédé selon l'une quelconque des revendications précédentes, selon lequel on ajoute à la réaction un co-facteur de formule générale R-O-PO₂H-CH₂-PO₃H₂, dans laquelle R est préférentiellement un atome d'hydrogène, un radical méthyle, éthyle ou propyle, et peut également être tout radical alcoyle, linéaire, branché ou cyclique, ou tout autre radical organique monovalent.

15. Procédé selon une quelconque des revendications précédentes, **caractérisé en ce que** l'étape de conversion est réalisée *in vitro,* en système acellulaire.

16. Procédé selon l'une quelconque des revendications 1 à 14 **caractérisé en ce que** l'étape de conversion est réalisée en présence d'un micro-organisme produisant ladite mévalonate diphosphate décarboxylase.

17. Procédé selon la revendication 16 **caractérisé par** l'utilisation d'un micro-organisme ayant la propriété naturelle ou artificielle de produire un ou plusieurs 3-hydroxy-alcanoates comme défini dans la revendication 1 de façon endogène, et exprimant ou surexprimant par ailleurs ladite mévalonate diphosphate décarboxylase, naturelle ou modifiée, afin de produire des alcènes terminaux directement à partir d'une source de carbone.

18. Procédé selon la revendication 17, où le micro-organisme est une bactérie de souche *Alcaligenes eutrophus* ou *Bacillus megaterium,* ou une bactérie, une levure ou un champignon recombinant de façon à surproduire un ou plusieurs 3-hydroxy-alcanoates comme défini dans la revendication 1.

19. Un procédé selon l'une des revendications 17 ou 18, où la source de carbone est le glucose ou tout autre hexose, le xylose ou tout autre pentose, le glycérol ou tout autre polyol, ou encore l'amidon, la cellulose, l'hémicellulose, un poly-3-hydroxy-alcanoate ou tout autre polymère, le procédé étant alors réalisé en présence d'un système pour dégrader ledit polymère en monomère, comme par exemple une enzyme adaptée (amylase, hémicellulase, cellulase, poly-3-hydroxy-alcanoase) et/ou des conditions chimiques particulières.

20. Procédé selon la revendication 17, **caractérisé par** l'utilisation d'un micro-organisme photosynthétique ayant la propriété naturelle ou artificielle de produire un ou plusieurs 3-hydroxy-alcanoates de façon endogène, et surexprimant par ailleurs la mévalonate diphosphate décarboxylase, naturelle ou modifiée, afin de produire des alcènes terminaux directement à partir de CO₂ présent en solution.

21. Procédé selon l'une quelconque des revendications 16 à 20, **caractérisé par** l'utilisation d'un premier micro-organisme permettant la transformation d'une source de carbone en 3-hydroxy-alcanoate comme défini dans la revendication 1, et d'une mévalonate diphosphate décarboxylase, isolée ou exprimée par un second micro-organisme, permettant la conversion dudit 3-hydroxy-alcanoate en alcène terminal.

22. Procédé selon l'une quelconque des revendications 1 à 14, **caractérisée par** l'utilisation d'une plante exprimant une mévalonate diphosphate décarboxylase, pour produire des alcènes terminaux par décarboxylation de 3-hydroxy-alcanoates comme défini dans la revendication 1.

23. Procédé selon la revendication 22, **caractérisé par** l'utilisation d'une plante ayant la propriété artificielle de produire de façon endogène un ou plusieurs 3-hydroxy-alcanoates comme défini dans la revendication 1.

24. Procédé selon l'une quelconque des revendications précédentes, comprenant une étape de collecte gazeuse des alcènes terminaux dégazant de la réaction.

25. Procédé selon une ou plusieurs des revendications précédente **caractérisé par le fait que** le procédé est mené en micro-aérophilie.

26. Utilisation d'une enzyme mévalonate diphosphate décarboxylase, ou d'un micro-organisme produisant une mévalonate diphosphate décarboxylase, pour la production de composés alcènes terminaux à partir de 3-hydroxy-alcanoates comme défini dans la revendication 1.

27. Utilisation d'une enzyme mévalonate diphosphate décarboxylase selon la revendication 26, **caractérisée en ce que** l'enzyme comprend tout ou partie de la séquence SEQ ID NO : 6 ou d'une séquence présentant au moins 15% d'homologie de séquence avec celle-ci.

28. Utilisation selon la revendication 27, dans laquelle l'enzyme comprend une séquence en acides aminés présentant au moins 50% ou au moins 80% ou au moins 90% d'homologie de séquence avec SEQ ID NO: 6.

29. Composition comprenant un micro-organisme produisant une mévalonate diphosphate décarboxylase, un milieu de culture approprié et un composé 3-hydroxy-alcanoate comme défini dans la revendication 1.

30. Une plante ou un micro-organisme ayant la propriété naturelle ou artificielle de produire un ou plusieurs 3-hydroxy-alcanoates comme défini dans la revendication 1 de façon endogène, et exprimant ou surexprimant par ailleurs une mévalonate diphosphate décarboxylase, naturelle ou modifiée, afin de produire des alcènes terminaux directement à partir d'une source de carbone.

## Patentansprüche

1. Verfahren zur Herstellung eines terminalen Alkens, **dadurch gekennzeichnet, dass** es einen Schritt der Umwandlung eines 3-Hydroxyalkanoats durch ein Enzym umfasst, das Mevalonatdiphosphatdecarboxylase-Aktivität aufweist, in dem das 3-Hydroxyalkanoat eine Verbindung der Formel
HO-CO-CH₂-C<R¹)(R²)-OH
oder
O⁻-CO-CH₂-C(R¹)(R²)-OH
ist, in der R¹ und R² unabhängig voneinander aus einem Wasserstoffatom, einem Methylrest und einem Ethylrest ausgewählt sind, und in dem die Umwandlung in Anwesenheit eines Co-Substrates erfolgt, wobei das Co-Substrat ATP, ein rNTP, ein dNTP oder ein Gemisch aus mehreren von diesen Molekülen, ein Polyphosphat oder Pyrophosphat ist.

2. Verfahren nach Anspruch 1, umfassend einen Schritt der Umwandlung von 3-Hydroxybutyrat in Propylen.

3. Verfahren nach Anspruch 1, umfassend einen Schritt der Umwandlung von 3-Hydroxyvalerat in 1-Butylen.

4. Verfahren nach Anspruch 1, umfassend einen Schritt der Umwandlung von 3-Hydroxy-3-methylbutyrat in Isobutylen.

5. Verfahren nach Anspruch 1, umfassend einen Schritt der Umwandlung von 3-Hydroxy-3-methylvalerat in Isoamylen.

6. Verfahren nach einem oder mehreren der vorangehenden Ansprüche, wobei das Enzym eine Aminosäuresequenz umfasst, die ausgewählt ist aus SEQ ID NO:1 bis 16, oder eine Sequenz umfasst, die mindestens 15% Sequenzhomologie mit einer dieser Sequenzen aufweist.

7. Verfahren nach Anspruch 6, wobei das Enzym eine Aminosäuresequenz umfasst, die mindestens 50% oder mindestens 80% oder mindestens 90% Sequenzhomologie mit einer der Sequenzen SEQ ID NO:1 bis 16 aufweist.

8. Verfahren nach einem der vorangehenden Ansprüche, **dadurch gekennzeichnet, dass** das Enzym die ganze oder einen Teil der Sequenz SEQ ID NO:6 oder einer Sequenz umfasst, die mindestens 15% Sequenzhomologie mit dieser aufweist.

9. Verfahren nach Anspruch 8, wobei das Enzym eine Aminosäuresequenz umfasst, die mindestens 50% oder mindestens 80% oder mindestens 90% Sequenzhomologie mit SEQ ID NO:6 aufweist.

10. Verfahren zur Herstellung von Isobutylen, **dadurch gekennzeichnet, dass** es einen Schritt der Umwandlung von 3-Hydroxy-3-methylbutyrat durch ein Enzym umfasst, das Mevalonatdiphosphatdecarboxylase-Aktivität aufweist.

11. Verfahren nach Anspruch 10, in dem das Enzym, das Mevalonatdiphosphatdecarboxylase-Aktivität aufweist, ein Enzym ist, das die ganze oder einen Teil der Sequenz SEQ ID NO:6 oder einer Sequenz umfasst, die mindestens 15% Sequenzhomologie mit dieser aufweist.

12. Verfahren nach Anspruch 11, in dem das Enzym eine Aminosäuresequenz umfasst, die mindestens 50% oder mindestens 80% oder mindestens 90% Sequenzhomologie mit der SEQ ID NO:6 aufweist.

13. Verfahren nach einem der vorangehenden Ansprüche, wobei der Reaktion ein Co-Faktor der allgemeinen Formel R-O-PO₂H-O-PO₃H₂ hinzugefügt wird, in der R ein Wasserstoffatom, ein Methylradikal, Ethylradikal oder Propylradikal ist und ebenfalls jedes lineare, verzweigte oder zyklische Alkylradikal oder jedes andere organische monovalente Radikal sein kann.

14. Verfahren nach einem der vorangehenden Ansprüche, wobei der Reaktion ein Co-Faktor der allgemeinen Formel R-O-PO₂H-CH₂-PO₃H₂ hinzugefügt wird, in der R bevorzugt ein Wasserstoffatom, ein Methylradikal, Ethylradikal oder Propylradikal ist und ebenfalls jedes lineare, verzweigte oder zyklische Alkylradikal oder jedes andere organische monovalente Radikal sein kann.

15. Verfahren nach einem der vorangehenden Ansprüche, **dadurch gekennzeichnet, dass** der Schritt der Umwandlung in einem azellulären System *in vitro* durchgeführt wird.

16. Verfahren nach einem der Ansprüche 1 bis 14, **dadurch gekennzeichnet, dass** der Schritt der Umwandlung in Anwesenheit eines Mikroorganismus durchgeführt wird, der die Mevalonatdiphosphatdecarboxylase herstellt.

17. Verfahren nach Anspruch 16, das durch die Verwendung eines Mikroorganismus gekennzeichnet ist, der die natürliche oder künstliche Eigenschaft hat, ein oder mehrere wie in Anspruch 1 definiertes/definierte 3-Hydroxyalkanoat/e endogen herzustellen, und der des Weiteren die natürliche oder modifizierte Mevalonatdiphosphatdecarboxylase exprimiert oder überexprimiert, um direkt aus einer Kohlenstoffquelle terminale Alkene herzustellen.

18. Verfahren nach Anspruch 17, wobei der Mikroorganismus ein Bakterium des Stammes *Alcaligenes eutrophus* oder *Bacillus megaterium* ist, oder ein rekombinantes Bakterium, eine rekombinante Hefe oder ein rekombinanter Pilz, die so modifiziert sind, dass sie ein oder mehrere wie in Anspruch 1 definiertes/definierte 3-Hydroxyalkanoat/e überproduzieren.

19. Verfahren nach einem der Ansprüche 17 oder 18, wobei die Kohlenstoffquelle Glucose oder jede andere Hexose, Xylose oder jede andere Pentose, Glycerol oder jedes andere Polyol, oder des Weiteren Stärke, Cellulose, Hemicellulose, ein Poly-3-hydroxyalkanoat oder jedes andere Polymer ist, wobei das Verfahren folglich in Anwesenheit eines Systems zum Abbau des Polymers in ein Monomer, wie beispielsweise ein geeignetes Enzym (Amylase, Hemicellulase, Cellulase, Poly-3-hydroxyalkanoase) und/oder unter spezifischen chemischen Bedingungen durchgeführt wird.

20. Verfahren nach Anspruch 17, das durch die Verwendung eines photosynthetischen Mikroorganismus gekennzeichnet ist, der die natürliche oder künstliche Eigenschaft hat, ein oder mehrere 3-Hydroxyalkanoat/e endogen herzustellen und der des Weiteren die natürliche oder veränderte Mevalonatdiphosphatdecarboxylase überexprimiert, um direkt aus in Lösung vorhandenem CO₂ terminale Alkene herzustellen.

21. Verfahren nach einem der Ansprüche 16 bis 20, das **dadurch gekennzeichnet ist, dass** ein erster Mikroorganismus verwendet wird, der die Umwandlung einer Kohlenstoffquelle in 3-Hydroxyalkanoat wie in Anspruch 1 definiert erlaubt, und dadurch dass eine Mevalonatdiphosphatdecarboxylase verwendet wird, die aus einem zweiten Mikroorganismus isoliert ist oder von diesem exprimiert wird, wodurch die Umwandlung des 3-Hydroxyalkanoats in ein terminales Alken ermöglicht wird.

22. Verfahren nach einem der Ansprüche 1 bis 14, das **dadurch gekennzeichnet ist, dass** eine Pflanze verwendet wird, die eine Mevalonatdiphosphatdecarboxylase exprimiert, um terminale Alkene durch Decarboxylierung von wie in Anspruch 1 definierten 3-Hydroxyalkanoaten herzustellen.

23. Verfahren nach Anspruch 22, das **dadurch gekennzeichnet ist, dass** eine Pflanze verwendet wird, die die künstliche Eigenschaft hat, ein oder mehrere wie in Anspruch 1 definierte/s 3-Hydroxyalkanoat/e endogen herzustellen.

24. Verfahren nach einem der vorangehenden Ansprüche, das einen Schritt des Gewinnens von terminalen Alkenen in Gasform umfasst, die bei der Reaktion ausgasen.

25. Verfahren nach einem oder mehreren der vorangehenden Ansprüche, das **dadurch gekennzeichnet ist, dass** es mikroaerophil durchgeführt wird.

26. Verwendung eines Mevalonatdiphosphatdecarboxylase-Enzyms oder eines Mikroorganismus, der eine Mevalonatdiphosphatdecarboxylase herstellt, zur Herstellung von terminalen Alkenverbindungen ausgehend von wie in Anspruch 1 definierten 3-Hydroxyalkanoaten.

27. Verwendung eines Mevalonatdiphosphatdecarboxylase-Enzyms nach Anspruch 26, **dadurch gekennzeichnet, dass** das Enzym die ganze oder einen Teil der Sequenz SEQ ID NO:6 oder einer Sequenz umfasst, die mindestens 15% Sequenzhomologie mit dieser aufweist.

28. Verwendung nach Anspruch 27, wobei das Enzym eine Aminosäuresequenz umfasst, die mindestens 50% oder mindestens 80% oder mindestens 90% Sequenzhomologie mit SEQ ID NO:6 aufweist.

29. Zusammensetzung, die einen Mikroorganismus, der eine Mevalonatdiphosphatdecarboxylase herstellt, ein geeignetes Kulturmedium und eine wie in Anspruch 1 definierte 3-Hydroxyalkanoatverbindung umfasst.

30. Pflanze oder Mikroorganismus, die/der die natürliche oder künstliche Eigenschaft aufweist, ein oder mehrere wie in Anspruch 1 definierte/s 3-Hydroxyalkanoat/e endogen herzustellen, und die/der des Weiteren eine natürliche oder veränderte Mevalonatdiphosphatdecarboxylase exprimiert oder überexprimiert, um direkt aus einer Kohlenstoffquelle terminale Alkene herzustellen.

## Claims

1. A method for the production of a terminal alkene, **characterized in that** it comprises a step of converting a 3-hydroxyalkanoate by an enzyme which has mevalonate diphosphate decarboxylase activity, in which the 3-hydroxyalkanoate is a compound of the formula
HO-CO-CH₂-C(R¹)(R²)-OH
or
O⁻-CO-CH₂-C(R¹)(R²)-OH
in which R¹ and R² are independently selected from a hydrogen atom, a methyl group and an ethyl group, and in which the conversion takes place in the presence of a co-substrate, wherein the co-substrate is ATP, an rNTP, a dNTP or a mixture of several of said molecules, a polyphosphate or pyrophosphate.

2. The method according to claim 1, comprising a step of converting 3-hydroxybutyrate to propylene.

3. The method according to claim 1, comprising a step of converting 3-hydroxyvalerate to 1-butylene.

4. The method according to claim 1, comprising a step of converting 3-hydroxy-3-methylbutyrate to isobutylene.

5. The method according to claim 1, comprising a step of converting 3-hydroxy-3-methylvalerate to isoamylene.

6. The method according to one or more of the preceding claims, wherein the enzyme comprises an amino acid sequence selected from SEQ ID NOs:1-16, or a sequence having at least 15% sequence homology with one of said sequences.

7. The method according to claim 6, wherein the enzyme comprises an amino acid sequence having at least 50% or at least 80% or at least 90% sequence homology with one of the sequences SEQ ID NO:1-16.

8. The method according to any of the preceding claims, **characterized in that** the enzyme comprises all or part of the sequence SEQ ID NO:6 or of a sequence having at least 15% sequence homology to said sequence.

9. The method according to claim 8, wherein the enzyme comprises an amino acid sequence having at least 50% or at least 80% or at least 90% sequence homology with SEQ ID NO:6.

10. A method for producing isobutylene, **characterized in that** it comprises a step of converting 3-hydroxy-3-methylbutyrate by an enzyme having mevalonate diphosphate decarboxylase activity.

11. The method according to claim 10, wherein the enzyme having mevalonate diphosphate decarboxylase activity is an enzyme comprising all or part of the sequence SEQ ID NO:6 or of a sequence having at least 15% sequence homology with said sequence.

12. The method according to claim 11, wherein the enzyme comprises an amino acid sequence having at least 50% or at least 80% or at least 90% sequence homology with SEQ ID NO:6.

13. The method according to any of the preceding claims, wherein a co-factor of the general formula R-O-PO₂H-O-PO₃H₂ is added to the reaction, wherein R is a hydrogen atom, a methyl radical, an ethyl radical or a propyl radical and can also be any linear, branched or cyclic alkyl radical or any other organic monovalent radical.

14. The method according to any of the preceding claims, wherein a co-factor of the general formula R-O-PO₂H-CH₂-PO₃H₂ is added to the reaction, wherein R preferably is a hydrogen atom, a methyl radical, a ethyl radical or a propyl radical and can also be any linear, branched or cyclic alkyl radical or any other organic monovalent radical.

15. The method according to any of the preceding claims, **characterized in that** the conversion step is carried out *in vitro* in a cell-free system.

16. The method according to any one of claims 1 to 14, **characterized in that** the conversion step is carried out in the presence of a microorganism which produces the mevalonate diphosphate decarboxylase.

17. The method according to claim 16, **characterized by** the use of a microorganism which has the natural or artificial property of endogenously producing one or more 3-hydroxyalkanoate(s) as defined in claim 1 and which further expresses or overexpresses natural or modified mevalonate diphosphate decarboxylase so as to produce terminal alkenes directly from a carbon source.

18. The method according to claim 17, wherein the microorganism is a bacterium of the strain *Alcaligenes eutrophus* or *Bacillus megaterium,* or a recombinant bacterium, a recombinant yeast or a recombinant fungus which is modified in such a manner that it overproduces one or more 3-hydroxyalkanoate(s) as defined in claim 1.

19. The method according to any one of claims 17 or 18, wherein the carbon source is glucose or any other hexose, xylose or any other pentose, glycerol or any other polyol, or further starch, cellulose, hemicellulose, a poly-3-hydroxyalkanoate or any other polymer, wherein the method is then being carried out in the presence of a system for the degradation of the polymer into a monomer such as for example a suitable enzyme (amylase, hemicellulase, cellulase, poly-3-hydroxyalkanoase) and/or under specific chemical conditions.

20. The method according to claim 17, which is **characterized by** the use of a photosynthetic microorganism which has the natural or artificial property of endogenously producing one or more 3-hydroxyalkanoate(s) and which further overexpresses natural or modified mevalonate diphosphate decarboxylase so as to produce terminal alkenes directly from CO₂ present in solution.

21. The method according to any one of claims 16 to 20, which is **characterized in that** a first microorganism is used allowing the conversion of a carbon source to 3-hydroxyalkanoate as defined in claim 1 and **in that** a mevalonate diphosphate decarboxylase is used which is isolated from or expressed by a second microorganism, allowing the conversion of the 3-hydroxyalkanoate to a terminal alkene.

22. The method according to any one of claims 1 to 14, which is **characterized in that** a plant is used which expresses a mevalonate diphosphate decarboxylase for producing terminal alkenes by decarboxylation of 3-hydroxyalkanoates as defined in claim 1.

23. The method according to claim 22, which is **characterized in that** a plant is used which has the artificial property of endogenously producing one or more 3-hydroxyalkanoate(s) as defined in claim 1.

24. The method according to any of the preceding claims, which comprises a step of collecting terminal alkenes in gas form, which degas from the reaction.

25. The method according to one or more of the preceding claims, which is **characterized in that** it is carried out in microaerophilic conditions.

26. Use of a mevalonate diphosphate decarboxylase enzyme or of a microorganism which produces a mevalonate diphosphate decarboxylase for the production of terminal alkene compounds from 3-hydroxyalkanoates as defined in claim 1.

27. The use of a mevalonate diphosphate decarboxylase enzyme according to claim 26, **characterized in that** the enzyme comprises all or part of the sequence SEQ ID NO:6 or of a sequence having at least 15% sequence homology with that sequence.

28. The use according to claim 27, wherein the enzyme comprises an amino acid sequence having at least 50% or at least 80% or at least 90% sequence homology with SEQ ID NO:6.

29. A composition comprising a microorganism which produces a mevalonate diphosphate decarboxylase, a suitable culture medium and a 3-hydroxyalkanoate compound as defined in claim 1.

30. A plant or microorganism having the natural or artificial property of endogenously producing one or more 3-hydroxyalkanoate(s) as defined in claim 1 and which further expresses or overexpresses a natural or modified mevalonate diphosphate decarboxylase so as to produce terminal alkenes directly from a carbon source.


### SEQUENCE LISTING

<110> Marlière, Philippe
<120> Production d'alcènes par décarboxylation d'acides 3-hydroxy-alcanoïques
<130> B742PC
<160> 20
<170> PatentIn version 3.3
<210> 1
   <211> 400
   <212> PRT
   <213> Homo sapiens
<400> 1
   Met Ala Ser Glu Lys Pro Leu Ala Ala Val Thr Cys Thr Ala Pro Val
   1               5                   10                  15
   Asn Ile Ala Val Ile Lys Tyr Trp Gly Lys Arg Asp Glu Glu Leu Val
               20                  25                  30
   Leu Pro Ile Asn Ser Ser Leu Ser Val Thr Leu His Gln Asp Gln Leu
           35                  40                  45
   Lys Thr Thr Thr Thr Ala Val Ile Ser Lys Asp Phe Thr Glu Asp Arg
       50                  55                  60
   Ile Trp Leu Asn Gly Arg Glu Glu Asp Val Gly Gln Pro Arg Leu Gln
   65                  70                  75                  80
   Ala Cys Leu Arg Glu Ile Arg Cys Leu Ala Arg Lys Arg Arg Asn Ser
                   85                  90                  95
   Arg Asp Gly Asp Pro Leu Pro Ser Ser Leu Ser Cys Lys Val His Val
               100                 105                 110
   Ala Ser Val Asn Asn Phe Pro Thr Ala Ala Gly Leu Ala Ser Ser Ala
           115                 120                 125
   Ala Gly Tyr Ala Cys Leu Ala Tyr Thr Leu Ala Arg Val Tyr Gly Val
       130                 135                 140
   Glu Ser Asp Leu Ser Glu Val Ala Arg Arg Gly Ser Gly Ser Ala Cys
   145                 150                 155                 160
   Arg Ser Leu Tyr Gly Gly Phe Val Glu Trp Gln Met Gly Glu Gln Ala
                   165                 170                 175
   Asp Gly Lys Asp Ser Ile Ala Arg Gln Val Ala Pro Glu Ser His Trp
                   180                 185                 190
   Pro Glu Leu Arg Val Leu Ile Leu Val Val Ser Ala Glu Lys Lys Leu
           195                 200                 205
   Thr Gly Ser Thr Val Gly Met Arg Ala Ser Val Glu Thr Ser Pro Leu
       210                 215                 220
   Leu Arg Phe Arg Ala Glu Ser Val Val Pro Ala Arg Met Ala Glu Met
   225                 230                 235                 240
   Ala Arg Cys Ile Arg Glu Arg Asp Phe Pro Ser Phe Ala Gln Leu Thr
                   245                 250                 255
   Met Lys Asp Ser Asn Gln Phe His Ala Thr Cys Leu Asp Thr Phe Pro
               260                 265                 270
   Pro Ile Ser Tyr Leu Asn Ala Ile Ser Trp Arg Ile Ile His Leu Val
           275                 280                 285
   His Arg Phe Asn Ala His His Gly Asp Thr Lys Val Ala Tyr Thr Phe
       290                 295                 300
   Asp Ala Gly Pro Asn Ala Val Ile Phe Thr Leu Asp Asp Thr Val Ala
   305                 310                 315                 320
   Glu Phe Val Ala Ala Val Trp His Gly Phe Pro Pro Gly Ser Asn Gly
                   325                 330                 335
   Asp Thr Phe Leu Lys Gly Leu Gln Val Arg Pro Ala Pro Leu Ser Ala
               340                 345                 350
   Glu Leu Gln Ala Ala Leu Ala Met Glu Pro Thr Pro Gly Gly Val Lys
           355                 360                 365
   Tyr Ile Ile Val Thr Gln Val Gly Pro Gly Pro Gln Ile Leu Asp Asp
       370                 375                 380
   Pro Cys Ala His Leu Leu Gly Pro Asp Gly Leu Pro Lys Pro Ala Ala
   385                 390                 395                 400
<210> 2
   <211> 396
   <212> PRT
   <213> Saccharomyces cerevisiae
<400> 2
   Met Thr Val Tyr Thr Ala Ser Val Thr Ala Pro Val Asn Ile Ala Thr
   1               5                   10                  15
   Leu Lys Tyr Trp Gly Lys Arg Asp Thr Lys Leu Asn Leu Pro Thr Asn
   20 25 30
   Ser Ser Ile Ser Val Thr Leu Ser Gln Asp Asp Leu Arg Thr Leu Thr
   35 40 45
   Ser Ala Ala Thr Ala Pro Glu Phe Glu Arg Asp Thr Leu Trp Leu Asn
   50 55 60
   Gly Glu Pro His Ser Ile Asp Asn Glu Arg Thr Gln Asn Cys Leu Arg
   65 70 75 80
   Asp Leu Arg Gln Leu Arg Lys Glu Met Glu Ser Lys Asp Ala Ser Leu
   85 90 95
   Pro Thr Leu Ser Gln Trp Lys Leu His Ile Val Ser Glu Asn Asn Phe
   100 105 110
   Pro Thr Ala Ala Gly Leu Ala Ser Ser Ala Ala Gly Phe Ala Ala Leu
   115 120 125
   Val Ser Ala Ile Ala Lys Leu Tyr Gln Leu Pro Gln Ser Thr Ser Glu
   130 135 140
   Ile Ser Arg Ile Ala Arg Lys Gly Ser Gly Ser Ala Cys Arg Ser Leu
   145 150 155 160
   Phe Gly Gly Tyr Val Ala Trp Glu Met Gly Lys Ala Glu Asp Gly His
   165 170 175
   Asp Ser Met Ala Val Gln Ile Ala Asp Ser Ser Asp Trp Pro Gln Met
   180 185 190
   Lys Ala Cys Val Leu Val Val Ser Asp Ile Lys Lys Asp Val Ser Ser
   195 200 205
   Thr Gln Gly Met Gln Leu Thr Val Ala Thr Ser Glu Leu Phe Lys Glu
   210 215 220
   Arg Ile Glu His Val Val Pro Lys Arg Phe Glu Val Met Arg Lys Ala
   225 230 235 240
   Ile Val Glu Lys Asp Phe Ala Thr Phe Ala Lys Glu Thr Met Met Asp
   245 250 255
   Ser Asn Ser Phe His Ala Thr Cys Leu Asp Ser Phe Pro Pro Ile Phe
   260 265 270
   Tyr Met Asn Asp Thr Ser Lys Arg Ile Ile Ser Trp Cys His Thr Ile
           275                 280                 285
   Asn Gln Phe Tyr Gly Glu Thr Ile Val Ala Tyr Thr Phe Asp Ala Gly
       290                 295                 300
   Pro Asn Ala Val Leu Tyr Tyr Leu Ala Glu Asn Glu Ser Lys Leu Phe
   305                 310                 315                 320
   Ala Phe Ile Tyr Lys Leu Phe Gly Ser Val Pro Gly Trp Asp Lys Lys
                   325                 330                 335
   Phe Thr Thr Glu Gln Leu Glu Ala Phe Asn His Gln Phe Glu Ser Ser
               340                 345                 350
   Asn Phe Thr Ala Arg Glu Leu Asp Leu Glu Leu Gln Lys Asp Val Ala
           355                 360                 365
   Arg Val Ile Leu Thr Gln Val Gly Ser Gly Pro Gln Glu Thr Asn Glu
       370                 375                 380
   Ser Leu Ile Asp Ala Lys Thr Gly Leu Pro Lys Glu
   385                 390                 395
<210> 3
   <211> 404
   <212> PRT
   <213> Aspergillus niger
<400> 3
   Met Ala Ala Ser Ala Asp Ser Gln Val Phe Arg Ala Thr Thr Thr Ala
   1               5                   10                  15
   Pro Val Asn Ile Ala Val Ile Lys Tyr Trp Gly Lys Arg Asp Ala Val
               20                  25                  30
   Leu Asn Leu Pro Thr Asn Ser Ser Leu Ser Val Thr Leu Ser Gln Arg
           35                  40                  45
   Ser Leu Arg Thr Leu Thr Thr Ala Ser Cys Ala Pro Phe Tyr Pro Ala
       50                  55                  60
   Lys Asp Glu Leu Thr Leu Asn Gly Lys Pro Gln Asp Ile Gln Ser Ser
   65                  70                  75                  80
   Lys Arg Thr Leu Ala Cys Leu Ala Ser Leu Arg Ala His Arg Arg Glu
                   85                  90                  95
   Leu Glu Asp Ala Asn Pro Ser Leu Pro Lys Leu Ser Ser Phe Pro Leu
               100                 105                 110
   Arg Ile Val Ser Glu Asn Asn Phe Pro Thr Ala Ala Gly Leu Ala Ser
           115                 120                 125
   Ser Ala Ala Gly Phe Ala Ala Leu Val Arg Ala Val Ala Asp Leu Tyr
       130                 135                 140
   Gln Leu Pro Gln Ser Pro Arg Asp Leu Ser Arg Ile Ala Arg Gln Gly
   145                 150                 155                 160
   Ser Gly Ser Ala Cys Arg Ser Leu Met Gly Gly Tyr Val Ala Trp Arg
                   165                 170                 175
   Ala Gly Ser Leu Glu Asp Gly Ser Asp Ser Leu Ala Glu Glu Val Ala
               180                 185                 190
   Pro Gln Ser His Trp Pro Glu Met Arg Ala Leu Ile Leu Val Val Ser
           195                 200                 205
   Ala Ala Lys Lys Asp Val Pro Ser Thr Glu Gly Met Gln Thr Thr Val
       210                 215                 220
   Ala Thr Ser Asn Leu Phe Ala Thr Arg Ala Ser Thr Val Val Pro Glu
   225                 230                 235                 240
   Arg Met Ala Ala Ile Glu Thr Ala Ile Gln Asn Arg Asp Phe Pro Ala
                   245                 250                 255
   Phe Ala Glu Ile Thr Met Arg Asp Ser Asn Ser Phe His Ala Thr Cys
               260                 265                 270
   Leu Asp Ser Trp Pro Pro Ile Phe Tyr Met Asn Asp Val Ser Arg Ala
           275                 280                 285
   Ala Val Arg Leu Val His Asp Ile Asn Arg Ala Ile Gly Arg Thr Val
       290                 295                 300
   Cys Ala Tyr Thr Tyr Asp Ala Gly Pro Asn Ala Val Ile Tyr Tyr Leu
   305                 310                 315                 320
   Glu Lys Asp Thr Glu Leu Val Ala Gly Thr Val Lys Ala Ile Leu Gly
                   325                 330                 335
   Glu Lys Thr Glu Gly Trp Glu Gly Pro Phe Tyr Thr Pro Leu Lys Asp
               340                 345                 350
   Val Thr Thr Pro Gly Val Ser Leu Asp Glu Ile Asp Pro Arg Thr Val
           355                 360                 365
   Glu Ser Leu Lys Asp Gly Val Ser Arg Val Ile Leu Thr Gly Val Gly
   370 375 380
   Glu Gly Pro Ile Ser Val Asp Gln His Leu Val Ser Glu Lys Gly Asp
   385 390 395 400
   Ile Leu Ser Ala
<210> 4
   <211> 325
   <212> PRT
   <213> Lactobacillus plantarum
<400> 4
   Met Lys Thr Val Thr Ala Lys Ala His Thr Asn Ile Ala Leu Val Lys
   1               5                   10                  15
   Tyr Trp Gly Lys Lys Asp Ala Ala Leu Met Leu Pro Gln Asn Gly Ser
               20                  25                  30
   Ile Ser Leu Thr Leu Asp His Phe Tyr Thr Gln Thr Ser Val Thr Phe
           35                  40                  45
   Asp Glu His Leu Asp Thr Asp Gln Ile Tyr Phe Asn His Gln His Leu
       50                  55                  60
   Pro Thr Gly Lys Ser Ala Arg Ile Ser Gln Phe Leu Asp Leu Ile Arg
   65                  70                  75                  80
   Gln Arg Ser Gly Gln Thr Asn Tyr Ala Thr Val Lys Thr Glu Asn His
                   85                  90                  95
   Val Pro Thr Ser Ala Gly Leu Ala Ser Ser Ala Ser Gly Phe Ala Ala
               100                 105                 110
   Leu Ala Gly Ala Ala Ser Arg Ala Ala Gly Leu Gln Leu Asp Ala Ala
           115                 120                 125
   Asp Leu Ser Arg Leu Ala Arg Arg Gly Ser Gly Ser Ala Thr Arg Ser
       130                 135                 140
   Ile Phe Gly Gly Phe Val Glu Trp His Ala Gly His Asp Asp Gln Ser
   145                 150                 155                 160
   Ser Tyr Ala Glu Val Leu Gln Asp Pro Val Asp Trp Asp Ile Gln Met
                   165                 170                 175
   Ile Ala Val Val Leu Lys Ala Thr Lys Lys Thr Ile Ser Ser Thr Asp
                   180                 185                 190
   Gly Met Ala Arg Val Val Ala Thr Ser Pro Tyr Tyr Pro Ala Trp Ile
           195                 200                 205
   Thr Thr Ala Glu Thr Asp Leu Lys Arg Met Arg Gln Ala Ile Ala Asp
       210                 215                 220
   Arg Asp Leu Thr Thr Val Gly Gln Ile Ala Glu Thr Asn Ala Met Arg
   225                 230                 235                 240
   Met His Ala Leu Asn Leu Ser Ala Glu Pro Ala Phe Asn Tyr Phe Thr
                   245                 250                 255
   Ala Asp Thr Leu Thr Ala Ile Gln Ala Val Asn Asp Leu Arg Ser His
               260                 265                 270
   Gly Ile Asn Cys Tyr Tyr Thr Leu Asp Ala Gly Pro Asn Val Lys Ile
           275                 280                 285
   Ile Cys Ala Gly Gln Asp Thr Asp Thr Ile Met Thr Gly Leu Gln Gln
       290                 295                 300
   His Phe Asp Ala Asp Gln Leu Ile Val Ala Lys Pro Gly Pro Gly Ile
   305                 310                 315                 320
   Thr Ile Thr Glu Lys
                   325
<210> 5
   <211> 314
   <212> PRT
   <213> Streptococcus pyrogenes
<400> 5
   Met Asp Pro Asn Val Ile Thr Val Thr Ser Tyr Ala Asn Ile Ala Ile
   1               5                   10                  15
   Ile Lys Tyr Trp Gly Lys Glu Asn Gln Ala Lys Met Ile Pro Ser Thr
               20                  25                  30
   Ser Ser Ile Ser Leu Thr Leu Glu Asn Met Phe Thr Thr Thr Ser Val
           35                  40                  45
   Ser Phe Leu Pro Asp Thr Ala Thr Ser Asp Gln Phe Tyr Ile Asn Gly
       50                  55                  60
   Ile Leu Gln Asn Asp Glu Glu His Thr Lys Ile Ser Ala Ile Ile Asp
   65                  70                  75                  80
   Gln Phe Arg Gln Pro Gly Gln Ala Phe Val Lys Met Glu Thr Gln Asn
                   85                  90                  95
   Asn Met Pro Thr Ala Ala Gly Leu Ser Ser Ser Ser Ser Gly Leu Ser
               100                 105                 110
   Ala Leu Val Lys Ala Cys Asp Gln Leu Phe Asp Thr Gln Leu Asp Gln
           115                 120                 125
   Lys Ala Leu Ala Gln Lys Ala Lys Phe Ala Ser Gly Ser Ser Ser Arg
       130                 135                 140
   Ser Phe Phe Gly Pro Val Ala Ala Trp Asp Lys Asp Ser Gly Ala Ile
   145                 150                 155                 160
   Tyr Lys Val Glu Thr Asp Leu Lys Met Ala Met Ile Met Leu Val Leu
                   165                 170                 175
   Asn Ala Ala Lys Lys Pro Ile Ser Ser Arg Glu Gly Met Lys Leu Cys
               180                 185                 190
   Arg Asp Thr Ser Thr Thr Phe Asp Gln Trp Val Glu Gln Ser Ala Ile
           195                 200                 205
   Asp Tyr Gln His Met Leu Thr Tyr Leu Lys Thr Asn Asn Phe Glu Lys
       210                 215                 220
   Val Gly Gln Leu Thr Glu Ala Asn Ala Leu Ala Met His Ala Thr Thr
   225                 230                 235                 240
   Lys Thr Ala Asn Pro Pro Phe Ser Tyr Leu Thr Lys Glu Ser Tyr Gln
                   245                 250                 255
   Ala Met Glu Ala Val Lys Glu Leu Arg Gln Glu Gly Phe Ala Cys Tyr
               260                 265                 270
   Phe Thr Met Asp Ala Gly Pro Asn Val Lys Val Leu Cys Leu Glu Lys
           275                 280                 285
   Asp Leu Ala Gln Leu Ala Glu Arg Leu Gly Lys Asn Tyr Arg Ile Ile
       290                 295                 300
   Val Ser Lys Thr Lys Asp Leu Pro Asp Val
   305                 310
<210> 6
   <211> 324
   <212> PRT
   <213> Picrophilus torridus
<400> 6
   Met Glu Asn Tyr Asn Val Lys Thr Arg Ala Phe Pro Thr Ile Gly Ile
   1               5                   10                  15
   Ile Leu Leu Gly Gly Ile Ser Asp Lys Lys Asn Arg Ile Pro Leu His
               20                  25                  30
   Thr Thr Ala Gly Ile Ala Tyr Thr Gly Ile Asn Asn Asp Val Tyr Thr
           35                  40                  45
   Glu Thr Lys Leu Tyr Val Ser Lys Asp Glu Lys Cys Tyr Ile Asp Gly
       50                  55                  60
   Lys Glu Ile Asp Leu Asn Ser Asp Arg Ser Pro Ser Lys Val Ile Asp
   65                  70                  75                  80
   Lys Phe Lys His Glu Ile Leu Met Arg Val Asn Leu Asp Asp Glu Asn
                   85                  90                  95
   Asn Leu Ser Ile Asp Ser Arg Asn Phe Asn Ile Leu Ser Gly Ser Ser
               100                 105                 110
   Asp Ser Gly Ala Ala Ala Leu Gly Glu Cys Ile Glu Ser Ile Phe Glu
           115                 120                 125
   Tyr Asn Ile Asn Ile Phe Thr Phe Glu Asn Asp Leu Gln Arg Ile Ser
       130                 135                 140
   Glu Ser Val Gly Arg Ser Leu Tyr Gly Gly Leu Thr Val Asn Tyr Ala
   145                 150                 155                 160
   Asn Gly Arg Glu Ser Leu Thr Glu Pro Leu Leu Glu Pro Glu Ala Phe
                   165                 170                 175
   Asn Asn Phe Thr Ile Ile Gly Ala His Phe Asn Ile Asp Arg Lys Pro
               180                 185                 190
   Ser Asn Glu Ile His Glu Asn Ile Ile Lys His Glu Asn Tyr Arg Glu
           195                 200                 205
   Arg Ile Lys Ser Ala Glu Arg Lys Ala Lys Lys Leu Glu Glu Leu Ser
       210                 215                 220
   Arg Asn Ala Asn Ile Lys Gly Ile Phe Glu Leu Ala Glu Ser Asp Thr
   225                 230                 235                 240
   Val Glu Tyr His Lys Met Leu His Asp Val Gly Val Asp Ile Ile Asn
                   245                 250                 255
   Asp Arg Met Glu Asn Leu Ile Glu Arg Val Lys Glu Met Lys Asn Asn
   260 265 270
   Phe Trp Asn Ser Tyr Ile Val Thr Gly Gly Pro Asn Val Phe Val Ile
   275 280 285
   Thr Glu Lys Lys Asp Val Asp Lys Ala Met Glu Gly Leu Asn Asp Leu
   290 295 300
   Cys Asp Asp Ile Arg Leu Leu Lys Val Ala Gly Lys Pro Gln Val Ile
   305 310 315 320
   Ser Lys Asn Phe
<210> 7
   <211> 319
   <212> PRT
   <213> Lactobacillus delbrueckii subsp. bulgaricus
<400> 7
   Met Ser Lys Thr Ala Arg Ala His Thr Asn Ile Ala Leu Ile Lys Tyr
   1 5 10 15
   Trp Gly Lys Lys Asp Ala Lys Leu Arg Leu Pro Leu Met Ser Ser Leu
   20 25 30
   Ser Met Thr Leu Asp Ala Phe Tyr Ser Asp Thr Lys Ile Ser Asp Ser
   35 40 45
   Glu Gln Met Ser Phe Lys Leu Asn Gly Gln Ala Val Ser Gly Pro Ala
   50 55 60
   Ala Asp Arg Val Phe Ala Tyr Leu Arg Ala Met Gln Asp Arg Phe Gly
   65 70 75 80
   Val Lys Gly Asn Leu Ala Val Glu Ser Val Asn Gln Val Pro Thr Ala
   85 90 95
   Ala Gly Leu Ala Ser Ser Ser Ser Ala Phe Ala Ala Met Ala Ala Ala
   100 105 110
   Phe Ala Asp His Tyr Gln Leu Gly Val Asp Arg Gln Glu Leu Ser Arg
   115 120 125
   Met Ala Arg Met Gly Ser Gly Ser Ala Ser Arg Ser Val Phe Gly Gly
   130 135 140
   Phe Ser Val Trp Gln Lys Gly Asp Ser Asp Gln Thr Ser Tyr Ala Tyr
   145                 150                 155                 160
   Pro Leu Asp Glu Glu Pro Asp Met Asp Leu Arg Leu Leu Ala Val Glu
                   165                 170                 175
   Ile Asn Asp Gln Glu Lys Lys Ile Ser Ser Thr Lys Gly Met Glu Met
               180                 185                 190
   Ser Lys Ser Ser Pro Phe Tyr Gln Val Trp Leu Asp Arg Asn Asp Ser
           195                 200                 205
   Glu Ile Lys Glu Met Glu Glu Ala Ile Lys Gln Ala Asp Phe Ser Lys
       210                 215                 220
   Leu Gly Ser Leu Ala Glu Leu Asn Ala Ser Glu Met His Thr Leu Thr
   225                 230                 235                 240
   Phe Thr Ala Val Pro Gly Phe Thr Tyr Phe Glu Pro Asn Thr Ile Lys
                   245                 250                 255
   Ala Ile Lys Leu Val Gln Asp Leu Arg Gln Gln Gly Leu Glu Cys Tyr
               260                 265                 270
   Tyr Thr Ile Asp Ala Gly Pro Asn Val Lys Val Leu Cys Gln Gly Lys
           275                 280                 285
   Asn Ser Lys Asp Ile Ile Asn Cys Phe Glu Ser Ser Phe Asp Arg Val
       290                 295                 300
   Lys Ile Ile Glu Ala Gly Phe Gly Pro Gly Val Thr Leu Leu Asp
   305                 310                 315
<210> 8
   <211> 324
   <212> PRT
   <213> Haloquadratum walsbyi
<400> 8
   Met Lys Ala Thr Ala Arg Ala His Pro Ile Gln Gly Leu Ile Lys Tyr
   1               5                   10                  15
   His Gly Met Arg Asp Ser Asp Lys Arg Tyr Pro Tyr His Asp Ser Ile
               20                  25                  30
   Ser Val Cys Thr Ala Pro Ser Ala Thr Thr Thr Thr Val Glu Phe Gln
           35                  40                  45
   Ser Asp Ala Ser Gly Asp Val Tyr Ile Ile Asp Asn Glu Arg Val Asp
       50                  55                  60
   Gly Arg Ala Ala Glu Arg Ile Asp Ala Val Val Glu His Val Arg Glu
   65                  70                  75                  80
   Arg Thr Gly Ile Arg Asp Pro Val Arg Leu Val Ser Thr Asn Ser Phe
                   85                  90                  95
   Pro Ser Asn Ile Gly Phe Gly Ser Ser Ser Ser Gly Phe Ala Ala Ala
               100                 105                 110
   Ala Met Ala Leu Val Thr Ala Ala Gly Glu Glu Leu Thr His Pro Glu
           115                 120                 125
   Ile Ser Thr Ile Ala Arg Arg Gly Ser Ser Ser Ala Ala Arg Ala Val
       130                 135                 140
   Thr Gly Ala Phe Ser Gln Leu Tyr Ser Gly Met Asn Asp Thr Asp Cys
   145                 150                 155                 160
   His Ala Glu Arg Ile Glu Thr Asp Leu Asp Ala Thr Val Arg Thr Val
                   165                 170                 175
   Ala Ala His Val Pro Ala Tyr Lys Glu Thr Glu Glu Ala His Arg Glu
               180                 185                 190
   Ala Ala Gln Ser His Met Phe Asp Ala Arg Leu Ala His Val His His
           195                 200                 205
   Gln Ile Asp Ala Met Arg Asp Ala Leu Tyr Asn Ala Asp Phe Asp Arg
       210                 215                 220
   Ile Phe Glu Leu Ala Glu His Asp Ser Leu Ser Leu Thr Ala Ala Thr
   225                 230                 235                 240
   Met Thr Gly Pro Ala Gly Trp Val Tyr Trp Gln Pro Gln Thr Ile Ala
                   245                 250                 255
   Val Phe Asn Thr Val Arg Glu Leu Arg Glu Arg Glu Ser Ile Pro Val
               260                 265                 270
   Tyr Phe Ser Thr Asp Thr Gly Ala Ser Val Tyr Val Asn Thr Thr Ala
           275                 280                 285
   Ala His Val Asp Thr Val Glu Ser Ala Ile Ser Asp Ile Gly Ile Asp
       290                 295                 300
   Thr Asp Ile Trp Thr Val Gly Gly Pro Ala Thr Val Leu Ser Ala Ser
   305                 310                 315                 320
   Asp Ser Leu Phe
<210> 9
   <211> 322
   <212> PRT
   <213> Lactobacillus salivarius subsp. salivarius
<400> 9
   Met Ser Asn His Ala Ala Ala Arg Ala His Thr Asn Ile Ala Leu Ile
   1               5                   10                  15
   Lys Tyr Trp Gly Lys Lys Asp Thr Glu Leu Ile Leu Pro Met Asn Asn
               20                  25                  30
   Ser Leu Ser Leu Thr Leu Asp His Phe Tyr Thr Asp Thr Ser Val Thr
           35                  40                  45
   Phe Asp Ser Ser Tyr Thr Lys Asp Thr Phe Ile Leu Asn Gly Lys Glu
       50                  55                  60
   Ile Pro Asn Glu Asn Val His Lys Phe Leu Asn Ile Val Arg Glu Lys
   65                  70                  75                  80
   Ala Gly Ile Ser Glu Phe Ala Lys Val Asn Ser Thr Asn His Val Pro
                   85                  90                  95
   Thr Thr Ala Gly Leu Ala Ser Ser Ala Ser Ala Phe Ala Ala Leu Ala
               100                 105                 110
   Ala Ala Ala Ser Lys Ala Ser Gly Met Asn Leu Ser Arg Arg Asp Leu
           115                 120                 125
   Ser Arg Leu Ala Arg Arg Gly Ser Gly Ser Ala Thr Arg Ser Ile Tyr
       130                 135                 140
   Gly Gly Phe Val Glu Trp Gln Ala Gly Asp Asn Asp Leu Asn Ser Tyr
   145                 150                 155                 160
   Ala Val Pro Phe Ile Glu Asn Val Ser Trp Asp Ile Lys Met Ile Ala
                   165                 170                 175
   Val Val Ile Asn Ser Lys Pro Lys Lys Ile Thr Ser Arg Ala Gly Met
               180                 185                 190
   Gln Thr Val Val Asn Thr Ser Pro Tyr Tyr Asn Ser Trp Ile Lys Glu
           195                 200                 205
   Ala Asn Arg Ser Ile Pro Leu Met Lys Glu Ala Ile Ser Lys Gln Asp
           210                 215                 220
   Phe Thr Thr Met Gly Glu Leu Ala Glu Glu Asn Ala Met Lys Met His
   225                 230                 235                 240
   Ala Leu Asn Leu Ser Ala His Pro His Phe Ser Tyr Phe Ser Pro Glu
                   245                 250                 255
   Ser Ile Gln Val Met Asn Leu Val Glu Glu Leu Arg Ser Met Gly Ile
               260                 265                 270
   Glu Cys Tyr Tyr Thr Met Asp Ala Gly Pro Asn Val Lys Ile Ile Cys
           275                 280                 285
   Leu Gly Lys Asp Thr Ala Ser Ile Thr Ser Phe Leu Gln Lys Asn Leu
       290                 295                 300
   Pro Asn Thr Glu Val Leu Val Ser Ser Ala Gly Pro Gly Val Gln Tyr
   305                 310                 315                 320
   Leu Asp
<210> 10
   <211> 314
   <212> PRT
   <213> Oenococcus oeni
<400> 10
   Met Ala Lys Val Arg Ala Tyr Thr Asn Ile Ala Leu Ile Lys Tyr Trp
   1               5                   10                  15
   Gly Lys Ser Asp Leu Asn Trp Asn Leu Pro Thr Ser Ser Ser Ile Gly
               20                  25                  30
   Leu Thr Leu Asp Arg Phe Tyr Thr Asp Thr Ser Val Glu Ile Asp Gln
           35                  40                  45
   Phe Ser Lys Lys Asp Phe Phe Gln Leu Asn Gly Gln Gln Ile Glu Gly
       50                  55                  60
   Pro Lys Ile Ser Lys Ile Ile Asn Phe Ile Arg Asn Ser Cys Gly Asn
   65                  70                  75                  80
   Lys Asn Phe Val Lys Val Ile Ser Glu Asn His Val Pro Thr Ser Ala
                   85                  90                  95
   Gly Leu Ala Ser Ser Ala Ser Ala Phe Ala Ala Leu Thr Lys Ala Ala
               100                 105                 110
   Asn Gln Ala Phe Gly Leu Glu Leu Asp Asn Arg Glu Leu Ser Lys Ile
           115                 120                 125
   Ala Arg Ile Gly Ser Gly Ser Ala Ser Arg Ser Ile Phe Gly Gly Phe
       130                 135                 140
   Ser Ile Trp His Lys Gly Gln Asn Lys Asp Asp Ser Phe Ala Glu Ser
   145                 150                 155                 160
   Ile Leu Asp Pro Val Asp Phe Asp Ile Arg Val Ile Asp Ile Leu Ala
                   165                 170                 175
   Asp Lys Arg Val Lys Lys Ile Ser Ser Ser Gln Gly Met Gln Leu Ala
               180                 185                 190
   Gln Thr Ser Pro Asn Tyr Asp Ser Trp Leu Lys Lys Asn Asp Arg Gln
           195                 200                 205
   Ile Asp Glu Met Leu Lys Ala Ile Ser Asp His Asp Leu Glu Lys Ile
       210                 215                 220
   Gly Leu Ile Ala Glu Thr Asn Ser Ala Ser Met His Glu Leu Asn Arg
   225                 230                 235                 240
   Thr Ala Lys Val Pro Phe Asp Tyr Phe Thr Glu Asn Thr Arg Glu Ile
                   245                 250                 255
   Ile Ala Glu Val Asp Gln Leu Tyr Lys Lys Gly Ile Leu Ala Phe Ala
               260                 265                 270
   Thr Val Asp Ala Gly Pro Asn Val Lys Val Ile Thr Asn Ser Glu Tyr
           275                 280                 285
   Gln Glu Lys Ile Ile Asn Val Leu Lys Glu Tyr Gly Glu Ile Leu Val
       290                 295                 300
   Gln Lys Pro Gly Arg Gly Val Ala Asn Val
   305                 310
<210> 11
   <211> 327
   <212> PRT
   <213> Pediococcus pentosaceus
<400> 11
   Met Asn Glu Lys His Gly Phe Ala Arg Ala His Thr Asn Ile Ala Leu
   1               5                   10                  15
   Leu Lys Tyr Trp Gly Lys Ile Asn Ser Asp Leu Ile Leu Pro Ala Asn
               20                  25                  30
   Asp Ser Ile Ser Leu Thr Leu Asp Lys Phe Tyr Thr Asp Thr Glu Val
           35                  40                  45
   Thr Phe Ser Asp Glu Tyr Thr Ser Asn Leu Phe Tyr Leu Asn His Gln
       50                  55                  60
   Leu Ile Asp Val Lys Lys Met Gln Arg Ile Asn Arg Val Leu Glu Ala
   65                  70                  75                  80
   Val Lys Ser Glu Phe Gly Tyr Gln Gly Phe Ala Lys Ile Glu Ser Glu
                   85                  90                  95
   Asn His Val Pro Thr Ala Ala Gly Leu Ala Ser Ser Ala Ser Gly Met
               100                 105                 110
   Ala Ala Leu Ala Gly Ala Ala Val Ser Ala Leu Gly Ser His Thr Asp
           115                 120                 125
   Leu Thr Asn Leu Ser Arg Leu Ala Arg Leu Gly Ser Gly Ser Ala Ser
       130                 135                 140
   Arg Ser Val Phe Gly Gly Ile Val His Trp His Arg Gly Tyr Asp His
   145                 150                 155                 160
   Gln Ser Ser Phe Ala Glu Gln Ile Val Ser Glu Asp Gln Ile Asp Leu
                   165                 170                 175
   Asn Met Val Thr Ile Val Ile Asp Arg Arg Gln Lys Lys Val Lys Ser
               180                 185                 190
   Thr Leu Gly Met Gln His Thr Ala Ser Thr Ser Pro Phe Tyr Pro Ala
           195                 200                 205
   Trp Val Glu Ala Thr Asn Gln Ala Ile Pro Glu Met Ile Ser Ala Val
       210                 215                 220
   Gln Asn Asn Asp Phe Thr Lys Ile Gly Glu Leu Ala Glu His Ser Ala
   225                 230                 235                 240
   Ala Met Met His Ala Thr Thr Leu Ser Ser Lys Pro Ala Phe Thr Tyr
                   245                 250                 255
   Phe Ala Pro Glu Thr Ile Gln Ala Ile Lys Leu Val Glu Gln Leu Arg
               260                 265                 270
   Glu Ser Gly Ile Glu Cys Tyr Tyr Thr Ile Asp Ala Gly Pro Asn Val
           275                 280                 285
   Lys Val Leu Cys Gln Ser Lys Asn Ile Thr Arg Val Lys Arg Phe Phe
   290 295 300
   Ala Ser Tyr Phe Asp Gln Asp Gln Leu Val Val Ala Lys Pro Gly Ser
   305 310 315 320
   Gly Ile Lys Phe Thr Lys Asn
   325
<210> 12
   <211> 315
   <212> PRT
   <213> Streptococcus gordonii
<400> 12
   Met Asp Arg Lys Pro Val Ser Val Lys Ser Tyr Ala Asn Ile Ala Ile
   1 5 10 15
   Val Lys Tyr Trp Gly Lys Lys Asp Ala Glu Lys Met Ile Pro Ser Thr
   20 25 30
   Ser Ser Ile Ser Leu Thr Leu Glu Asn Met Tyr Thr Glu Thr Gln Leu
   35 40 45
   Ser Pro Leu Pro Asp Thr Ala Thr Gly Asp Glu Phe Tyr Ile Asp Gly
   50 55 60
   Gln Leu Gln Ser Pro Ala Glu His Ala Lys Ile Ser Lys Ile Ile Asp
   65 70 75 80
   Arg Phe Arg Ser Pro Glu Asp Gly Phe Val Arg Val Asp Thr Ser Asn
   85 90 95
   Asn Met Pro Thr Ala Ala Gly Leu Ser Ser Ser Ser Ser Gly Leu Ser
   100 105 110
   Ala Leu Val Lys Ala Cys Asn Ala Tyr Phe Gln Thr Gly Tyr Gln Thr
   115 120 125
   Glu Glu Leu Ala Gln Leu Ala Lys Phe Ala Ser Gly Ser Ser Ala Arg
   130 135 140
   Ser Phe Phe Gly Pro Leu Ala Ala Trp Asp Lys Asp Ser Gly Ala Ile
   145 150 155 160
   Tyr Pro Val Lys Thr Asp Leu Lys Leu Ala Met Ile Met Leu Val Leu
   165 170 175
   His Asp Glu Lys Lys Pro Ile Ser Ser Arg Asp Gly Met Glu Leu Cys
   180 185 190
   Ala Lys Thr Ser Thr Ile Phe Pro Asp Trp Ile Ala Gln Ser Ala Leu
   195 200 205
   Asp Tyr Gln Ala Met Leu Gly Tyr Leu Gln Asp Asn Asp Phe Ala Lys
   210 215 220
   Val Gly Gln Leu Thr Glu Glu Asn Ala Leu Arg Met His Ala Thr Thr
   225 230 235 240
   Glu Lys Ala Tyr Pro Pro Phe Ser Tyr Leu Thr Glu Glu Ser Tyr Gln
   245 250 255
   Ala Met Asp Ala Val Arg Lys Leu Arg Glu Gln Gly Glu Arg Cys Tyr
   260 265 270
   Phe Thr Met Asp Ala Gly Pro Asn Val Lys Val Leu Cys Leu Glu Glu
   275 280 285
   Asp Leu Asp His Leu Ala Ala Ile Phe Glu Lys Asp Tyr Arg Leu Ile
   290 295 300
   Val Ser Lys Thr Lys Asp Leu Ser Asp Glu Ser
   305 310 315
<210> 13
   <211> 328
   <212> PRT
   <213> Dichelobacter nodosus
<400> 13
   Met His Ser Ala Thr Ala Phe Ala Pro Ala Asn Ile Ala Leu Ala Lys
   1               5                   10                  15
   Tyr Trp Gly Lys Arg Asp Ala Gln Leu Asn Leu Pro Thr Asn Gly Ser
               20                  25                  30
   Leu Ser Ile Ser Leu Ala His Leu Gly Thr Thr Thr Thr Ile Ser Ala
           35                  40                  45
   Gly Glu Arg Asp Gln Leu Tyr Cys Asp His Arg Leu Leu Pro Pro Asp
       50                  55                  60
   Thr Ala Phe Val Gln Lys Val Trp His Phe Ile Asp Phe Cys Gln Pro
   65                  70                  75                  80
   Lys Arg Pro Pro Leu Val Ile His Thr Gln Asn Asn Ile Pro Thr Ala
                   85                  90                  95
   Ala Gly Leu Ala Ser Ser Ala Ser Gly Phe Ala Ala Leu Thr Leu Ala
               100                 105                 110
   Leu Asn Asp Phe Phe Gln Trp Ser Leu Ser Arg Glu Gln Leu Ser Gln
           115                 120                 125
   Ile Ala Arg Arg Gly Ser Gly Ser Ala Cys Arg Ser Leu Trp Gln Gly
       130                 135                 140
   Phe Val Tyr Trp Gln Lys Gly Glu Lys Ala Asp Gly Ser Asp Cys Tyr
   145                 150                 155                 160
   Ala Arg Pro Ile Ala Ser Asp Trp Gln Asp Leu Arg Leu Gly Ile Ile
                   165                 170                 175
   Thr Ile Asp Ala Ala Ala Lys Lys Ile Ser Ser Arg Gln Ala Met Asn
               180                 185                 190
   His Thr Ala Ala Ser Ser Pro Leu Phe Ser Ser Trp Thr Gln Ala Ala
           195                 200                 205
   Glu Ala Asp Leu Lys Val Ile Tyr Gln Ala Val Leu Asp Arg Asp Phe
       210                 215                 220
   Leu Thr Leu Ala Gln Thr Ala Glu Ala Asn Ala Leu Met Met His Ala
   225                 230                 235                 240
   Ser Leu Leu Ala Ala Arg Pro Ala Ile Phe Tyr Trp Gln Pro Gln Thr
                   245                 250                 255
   Leu Ala Met Leu Gln Cys Ile Trp Gln Ala Arg Ala Glu Gly Leu Ala
               260                 265                 270
   Val Tyr Ala Thr Leu Asp Ala Gly Ala Asn Val Lys Leu Leu Tyr Arg
           275                 280                 285
   Ala Gln Asp Glu Ala Glu Ile Ala Ser Met Phe Pro Gln Ala Gln Leu
       290                 295                 300
   Ile Asn Pro Phe Gln Thr Val Thr Ser Ser Ala Arg His Thr Gly Glu
   305                 310                 315                 320
   Asp Ala Gln Lys Pro Ser Leu Lys
                   325
<210> 14
   <211> 317
   <212> PRT
   <213> Streptococcus pneumoniae
<400> 14
   Met Asp Arg Glu Pro Val Thr Val Arg Ser Tyr Ala Asn Ile Ala Ile
   1               5                   10                  15
   Ile Lys Tyr Trp Gly Lys Lys Lys Glu Lys Glu Met Val Pro Ala Thr
               20                  25                  30
   Ser Ser Ile Ser Leu Thr Leu Glu Asn Met Tyr Thr Glu Thr Thr Leu
           35                  40                  45
   Ser Pro Leu Pro Ala Asn Val Thr Ala Asp Glu Phe Tyr Ile Asn Gly
       50                  55                  60
   Gln Leu Gln Asn Glu Val Glu His Ala Lys Met Ser Lys Ile Ile Asp
   65                  70                  75                  80
   Arg Tyr Arg Pro Ala Gly Glu Gly Phe Val Arg Ile Asp Thr Gln Asn
                   85                  90                  95
   Asn Met Pro Thr Ala Ala Gly Leu Ser Ser Ser Ser Ser Gly Leu Ser
               100                 105                 110
   Ala Leu Val Lys Ala Cys Asn Ala Tyr Phe Lys Leu Gly Leu Asp Arg
           115                 120                 125
   Ser Gln Leu Ala Gln Glu Ala Lys Phe Ala Ser Gly Ser Ser Ser Arg
       130                 135                 140
   Ser Phe Tyr Gly Pro Leu Gly Ala Trp Asp Lys Asp Ser Gly Glu Ile
   145                 150                 155                 160
   Tyr Pro Val Glu Thr Asp Leu Lys Leu Ala Met Ile Met Leu Val Leu
                   165                 170                 175
   Glu Asp Lys Lys Lys Pro Ile Ser Ser Arg Asp Gly Met Lys Leu Cys
               180                 185                 190
   Val Glu Thr Ser Thr Thr Phe Asp Asp Trp Val Arg Gln Ser Glu Lys
           195                 200                 205
   Asp Tyr Gln Asp Met Leu Ile Tyr Leu Lys Glu Asn Asp Phe Ala Lys
       210                 215                 220
   Ile Gly Glu Leu Thr Glu Lys Asn Ala Leu Ala Met His Ala Thr Thr
   225                 230                 235                 240
   Lys Thr Ala Ser Pro Ala Phe Ser Tyr Leu Thr Asp Ala Ser Tyr Glu
                   245                 250                 255
   Ala Met Ala Phe Val Arg Gln Leu Arg Glu Lys Gly Glu Ala Cys Tyr
               260                 265                 270
   Phe Thr Met Asp Ala Gly Pro Asn Val Lys Val Phe Cys Gln Glu Lys
           275                 280                 285
   Asp Leu Glu His Leu Ser Glu Ile Phe Gly Gln Arg Tyr Arg Leu Ile
       290                 295                 300
   Val Ser Lys Thr Lys Asp Leu Ser Gln Asp Asp Cys Cys
   305                 310                 315
<210> 15
   <211> 314
   <212> PRT
   <213> Streptococcus pyogenes
<400> 15
   Met Asp Pro Asn Val Ile Thr Val Thr Ser Tyr Ala Asn Ile Ala Ile
   1               5                   10                  15
   Ile Lys Tyr Trp Gly Lys Glu Asn Gln Ala Lys Met Ile Pro Ser Thr
               20                  25                  30
   Ser Ser Ile Ser Leu Thr Leu Glu Asn Met Phe Thr Thr Thr Ser Val
           35                  40                  45
   Ser Phe Leu Pro Asp Thr Ala Thr Ser Asp Gln Phe Tyr Ile Asn Gly
       50                  55                  60
   Val Leu Gln Asn Asp Glu Glu His Thr Lys Ile Ser Ala Ile Ile Asp
   65                  70                  75                  80
   Gln Phe Arg Gln Pro Gly Gln Ala Phe Val Lys Met Glu Thr Gln Asn
                   85                  90                  95
   Asn Met Pro Thr Ala Ala Gly Leu Ser Ser Ser Ser Ser Gly Leu Ser
               100                 105                 110
   Ala Leu Val Lys Ala Cys Asp Gln Leu Phe Asn Thr Gln Leu Asp Gln
           115                 120                 125
   Lys Ala Leu Ala Gln Lys Ala Lys Phe Ala Ser Gly Ser Ser Ser Arg
       130                 135                 140
   Ser Phe Phe Gly Pro Val Ala Ala Trp Asp Lys Asp Ser Gly Ala Ile
   145                 150                 155                 160
   Tyr Lys Val Glu Thr Asp Leu Lys Met Ala Met Ile Met Leu Val Leu
                   165                 170                 175
   Asn Ala Ala Lys Lys Pro Ile Ser Ser Arg Glu Gly Met Lys Leu Cys
               180                 185                 190
   Arg Asp Thr Ser Thr Thr Phe Asp Glu Trp Val Glu Gln Ser Ala Ile
           195                 200                 205
   Asp Tyr Gln His Met Leu Thr Tyr Leu Lys Thr Asn Asn Phe Glu Lys
       210                 215                 220
   Val Gly Gln Leu Thr Glu Ala Asn Ala Leu Ala Met His Ala Thr Thr
   225                 230                 235                 240
   Lys Thr Ala Asn Pro Pro Phe Ser Tyr Leu Thr Lys Glu Ser Tyr Gln
                   245                 250                 255
   Ala Met Glu Ala Val Lys Glu Leu Arg Gln Glu Gly Phe Ala Cys Tyr
               260                 265                 270
   Phe Thr Met Asp Ala Gly Pro Asn Val Lys Val Leu Cys Leu Glu Lys
           275                 280                 285
   Asp Leu Ala Gln Leu Ala Glu Arg Leu Gly Lys Asn Tyr Arg Ile Ile
       290                 295                 300
   Val Ser Lys Thr Lys Asp Leu Pro Asp Val
   305                 310
<210> 16
   <211> 320
   <212> PRT
   <213> Lactobacillus acidophilus NCFM
<400> 16
   Met Lys Asn Thr Ala Arg Ala His Thr Asn Ile Ala Leu Ile Lys Tyr
   1               5                   10                  15
   Trp Gly Lys Ser Asp Pro Ile Leu Arg Leu Pro Leu Met Ser Ser Leu
               20                  25                  30
   Ser Met Thr Leu Asp Ala Phe Tyr Thr Asp Thr Leu Ile Glu Lys Thr
           35                  40                  45
   Asp Ala Lys Asn Glu Phe Tyr Leu Asn Gly Lys Arg Gln Asn Arg Gln
       50                  55                  60
   Ala Lys Lys Arg Val Phe Ser Tyr Leu Asp Thr Leu Lys Glu Lys Phe
   65                  70                  75                  80
   Gly Tyr Thr Asp Asn Leu Ile Val Lys Ser Thr Asn His Val Pro Thr
                   85                  90                  95
   Ser Ala Gly Leu Ala Ser Ser Ser Ser Ala Phe Ala Ala Leu Ala Ala
               100                 105                 110
   Ser Phe Cys Lys Leu Tyr Asn Leu Asp Val Asp Lys Thr Glu Leu Ser
           115                 120                 125
   Arg Leu Ala Arg Leu Gly Ser Gly Ser Ala Ser Arg Ser Ile Phe Gly
       130                 135                 140
   Gly Phe Ala Ile Trp Gln Lys Gly Asn Ser Asn Gln Ser Ser Tyr Ala
   145                 150                 155                 160
   Tyr Ala Leu Asp Glu Lys Pro Lys Met Asp Leu Gln Leu Leu Ala Val
                   165                 170                 175
   Glu Leu Asn Thr Glu Gln Lys Lys Ile Ser Ser Thr Lys Gly Met Lys
               180                 185                 190
   Asp Ala Gln Ser Ser Pro Phe Phe Ser Thr Trp Thr Asn Arg Asn Gln
           195                 200                 205
   Leu Glu Leu Asp Glu Met Ile Lys Ala Ile Lys Gln Asn Asp Phe Thr
       210                 215                 220
   Ala Leu Gly Ser Leu Ala Glu Leu Asn Ala Asn Glu Met His Ala Ile
   225                 230                 235                 240
   Asn Leu Thr Ala Gln Pro Glu Phe Thr Tyr Phe Met Pro Glu Thr Ile
                   245                 250                 255
   Arg Ala Ile Lys Leu Val Glu Asp Leu Arg Thr Lys Gly Ile Glu Cys
               260                 265                 270
   Tyr Tyr Thr Ile Asp Ala Gly Pro Asn Ile Lys Val Leu Cys Gln Leu
           275                 280                 285
   Lys Asn Arg Lys Glu Ile Ile Glu His Phe Glu Ser Val Phe Asn Asn
       290                 295                 300
   Val Asn Ile Val Ser Ala Ser Phe Gly Pro Gly Val Ile Tyr Leu Asp
   305                 310                 315                 320
<210> 17
   <211> 996
   <212> DNA
   <213> artificial sequence
<220>
   <223> Séquence optimisée de la MDP décarboxylase de P. torridus (AAT43941) incluant le His Tag
<220>
   <221> CDS
   <222> (1)..(996)
<400> 17
   atg cat cat cat cat cac cac gag aac tat aat gtt aaa acc cgt gca      48
   Met His His His His His His Glu Asn Tyr Asn Val Lys Thr Arg Ala
   1               5                   10                  15
   ttt ccg acc att ggt att att ctg ctg ggt ggc att agc gac aaa aaa      96
   Phe Pro Thr Ile Gly Ile Ile Leu Leu Gly Gly Ile Ser Asp Lys Lys
               20                  25                  30
   aac cgt att ccg ctg cat acc acc gca ggt att gca tat acc ggc atc     144
   Asn Arg Ile Pro Leu His Thr Thr Ala Gly Ile Ala Tyr Thr Gly Ile
           35                  40                  45
   aat aac gat gtg tac acc gaa acc aaa ctg tat gtg agc aaa gac gaa     192
   Asn Asn Asp Val Tyr Thr Glu Thr Lys Leu Tyr Val Ser Lys Asp Glu
       50                  55                  60
   aaa tgc tat atc gat ggc aaa gaa atc gat ctg aat agc gat cgt agc     240
   Lys Cys Tyr Ile Asp Gly Lys Glu Ile Asp Leu Asn Ser Asp Arg Ser
   65                  70                  75                  80
   ccg agc aaa gtg atc gat aaa ttc aaa cat gaa atc ctg atg cgt gtg     288
   Pro Ser Lys Val Ile Asp Lys Phe Lys His Glu Ile Leu Met Arg Val
                   85                  90                  95
   aat ctg gat gat gaa aac aac ctg agc att gat agc cgc aat ttt aac     336
   Asn Leu Asp Asp Glu Asn Asn Leu Ser Ile Asp Ser Arg Asn Phe Asn
               100                 105                 110
   att ctg agc ggt agc agc gat agc ggt gca gca gca ctg ggt gaa tgc     384
   Ile Leu Ser Gly Ser Ser Asp Ser Gly Ala Ala Ala Leu Gly Glu Cys
           115                 120                 125
   att gaa agc atc ttc gag tac aac atc aac atc ttc acc ttt gaa aat     432
   Ile Glu Ser Ile Phe Glu Tyr Asn Ile Asn Ile Phe Thr Phe Glu Asn
       130                 135                 140
   gat ctg cag cgt att agc gaa agc gtt ggt cgt agc ctg tat ggt ggt     480
   Asp Leu Gln Arg Ile Ser Glu Ser Val Gly Arg Ser Leu Tyr Gly Gly
   145                 150                 155                 160
   ctg acc gtt aat tat gca aat ggt cgt gaa agc ctg acc gaa ccg ctg     528
   Leu Thr Val Asn Tyr Ala Asn Gly Arg Glu Ser Leu Thr Glu Pro Leu
                   165                 170                 175
   ctg gaa ccg gaa gca ttt aac aac ttt acc atc atc ggt gcc cat ttt     576
   Leu Glu Pro Glu Ala Phe Asn Asn Phe Thr Ile Ile Gly Ala His Phe
               180                 185                 190
   aac att gat cgc aaa ccg agc aac gaa atc cac gaa aac atc atc aaa     624
   Asn Ile Asp Arg Lys Pro Ser Asn Glu Ile His Glu Asn Ile Ile Lys
           195                 200                 205
   cat gag aac tat cgc gaa cgt att aaa agc gca gag cgc aaa gca aaa        672
   His Glu Asn Tyr Arg Glu Arg Ile Lys Ser Ala Glu Arg Lys Ala Lys
       210                 215                 220
   aaa ctg gaa gaa ctg agc cgt aat gcc aac att aaa ggc att ttt gaa        720
   Lys Leu Glu Glu Leu Ser Arg Asn Ala Asn Ile Lys Gly Ile Phe Glu
   225                 230                 235                 240
   ctg gca gaa agc gat acc gtg gaa tat cat aaa atg ctg cat gat gtg        768
   Leu Ala Glu Ser Asp Thr Val Glu Tyr His Lys Met Leu His Asp Val
                   245                 250                 255
   ggc gtt gat att atc aat gac cgc atg gaa aat ctg att gaa cgc gtg        816
   Gly Val Asp Ile Ile Asn Asp Arg Met Glu Asn Leu Ile Glu Arg Val
               260                 265                 270
   aaa gag atg aaa aac aac ttc tgg aac agc tat att gtt acc ggt ggt        864
   Lys Glu Met Lys Asn Asn Phe Trp Asn Ser Tyr Ile Val Thr Gly Gly
           275                 280                 285
   ccg aat gtt ttt gtg atc acc gag aaa aaa gat gtg gat aaa gcc atg        912
   Pro Asn Val Phe Val Ile Thr Glu Lys Lys Asp Val Asp Lys Ala Met
       290                 295                 300
   gaa ggt ctg aat gat ctg tgt gat gat att cgt ctg ctg aaa gtt gca        960
   Glu Gly Leu Asn Asp Leu Cys Asp Asp Ile Arg Leu Leu Lys Val Ala
   305                 310                 315                 320
   ggt aaa ccg cag gtt atc agc aaa aac ttc taa tga                        996
   Gly Lys Pro Gln Val Ile Ser Lys Asn Phe
                   325                 330
<210> 18
   <211> 330
   <212> PRT
   <213> artificial sequence
<220>
   <223> Synthetic Construct
<400> 18
   Met His His His His His His Glu Asn Tyr Asn Val Lys Thr Arg Ala
   1               5                   10                  15
   Phe Pro Thr Ile Gly Ile Ile Leu Leu Gly Gly Ile Ser Asp Lys Lys
               20                  25                  30
   Asn Arg Ile Pro Leu His Thr Thr Ala Gly Ile Ala Tyr Thr Gly Ile
           35                  40                  45
   Asn Asn Asp Val Tyr Thr Glu Thr Lys Leu Tyr Val Ser Lys Asp Glu
       50                  55                  60
   Lys Cys Tyr Ile Asp Gly Lys Glu Ile Asp Leu Asn Ser Asp Arg Ser
   65                  70                  75                  80
   Pro Ser Lys Val Ile Asp Lys Phe Lys His Glu Ile Leu Met Arg Val
                   85                  90                  95
   Asn Leu Asp Asp Glu Asn Asn Leu Ser Ile Asp Ser Arg Asn Phe Asn
               100                 105                 110
   Ile Leu Ser Gly Ser Ser Asp Ser Gly Ala Ala Ala Leu Gly Glu Cys
           115                 120                 125
   Ile Glu Ser Ile Phe Glu Tyr Asn Ile Asn Ile Phe Thr Phe Glu Asn
       130                 135                 140
   Asp Leu Gln Arg Ile Ser Glu Ser Val Gly Arg Ser Leu Tyr Gly Gly
   145                 150                 155                 160
   Leu Thr Val Asn Tyr Ala Asn Gly Arg Glu Ser Leu Thr Glu Pro Leu
                   165                 170                 175
   Leu Glu Pro Glu Ala Phe Asn Asn Phe Thr Ile Ile Gly Ala His Phe
               180                 185                 190
   Asn Ile Asp Arg Lys Pro Ser Asn Glu Ile His Glu Asn Ile Ile Lys
           195                 200                 205
   His Glu Asn Tyr Arg Glu Arg Ile Lys Ser Ala Glu Arg Lys Ala Lys
       210                 215                 220
   Lys Leu Glu Glu Leu Ser Arg Asn Ala Asn Ile Lys Gly Ile Phe Glu
   225                 230                 235                 240
   Leu Ala Glu Ser Asp Thr Val Glu Tyr His Lys Met Leu His Asp Val
                   245                 250                 255
   Gly Val Asp Ile Ile Asn Asp Arg Met Glu Asn Leu Ile Glu Arg Val
               260                 265                 270
   Lys Glu Met Lys Asn Asn Phe Trp Asn Ser Tyr Ile Val Thr Gly Gly
           275                 280                 285
   Pro Asn Val Phe Val Ile Thr Glu Lys Lys Asp Val Asp Lys Ala Met
       290                 295                 300
   Glu Gly Leu Asn Asp Leu Cys Asp Asp Ile Arg Leu Leu Lys Val Ala
   305                 310                 315                 320
   Gly Lys Pro Gln Val Ile Ser Lys Asn Phe
                   325                 330
<210> 19
   <211> 993
   <212> DNA
   <213> Artificial sequence
<220>
   <223> Séquence native de la MDP décarboxylase de P. torridus (AAT43941) incluant le His Tag
<220>
   <221> CDS
   <222> (1)..(993)
<400> 19
   atg cat cat cac cat cac cat gaa aat tac aat gtt aag aca agg gcg      48
   Met His His His His His His Glu Asn Tyr Asn Val Lys Thr Arg Ala
   1               5                   10                  15
   ttc cca aca ata ggc ata ata ctg ctt ggt ggg atc tcg gat aaa aag      96
   Phe Pro Thr Ile Gly Ile Ile Leu Leu Gly Gly Ile Ser Asp Lys Lys
               20                  25                  30
   aac agg ata ccg ctg cat aca acg gca ggc ata gca tat act ggt ata     144
   Asn Arg Ile Pro Leu His Thr Thr Ala Gly Ile Ala Tyr Thr Gly Ile
           35                  40                  45
   aac aat gat gtt tac act gag aca aag ctt tat gta tca aaa gat gaa     192
   Asn Asn Asp Val Tyr Thr Glu Thr Lys Leu Tyr Val Ser Lys Asp Glu
       50                  55                  60
   aaa tgc tat att gat gga aag gaa att gat tta aat tca gat aga tca     240
   Lys Cys Tyr Ile Asp Gly Lys Glu Ile Asp Leu Asn Ser Asp Arg Ser
   65                  70                  75                  80
   cca tcg aag gtt att gat aaa ttc aag cat gaa ata ctt atg aga gta     288
   Pro Ser Lys Val Ile Asp Lys Phe Lys His Glu Ile Leu Met Arg Val
                   85                  90                  95
   aat ctt gat gat gaa aat aac ctt tca att gat tca agg aac ttt aat     336
   Asn Leu Asp Asp Glu Asn Asn Leu Ser Ile Asp Ser Arg Asn Phe Asn
               100                 105                 110
   ata tta agt ggc agc tca gat tct ggg gcc gct gca ctg gga gag tgc     384
   Ile Leu Ser Gly Ser Ser Asp Ser Gly Ala Ala Ala Leu Gly Glu Cys
           115                 120                 125
   ata gaa tca att ttt gaa tac aat ata aat ata ttt aca ttt gaa aac     432
   Ile Glu Ser Ile Phe Glu Tyr Asn Ile Asn Ile Phe Thr Phe Glu Asn
       130                 135                 140
   gat ctt cag agg ata tca gaa agt gtt gga aga agc ctt tac ggt ggt     480
   Asp Leu Gln Arg Ile Ser Glu Ser Val Gly Arg Ser Leu Tyr Gly Gly
   145                 150                 155                 160
   tta aca gta aac tat gcc aat ggc agg gaa tca tta aca gag cca tta     528
   Leu Thr Val Asn Tyr Ala Asn Gly Arg Glu Ser Leu Thr Glu Pro Leu
                   165                 170                 175
   ctt gag cct gag gca ttt aat aac ttt aca ata att ggt gca cat ttt     576
   Leu Glu Pro Glu Ala Phe Asn Asn Phe Thr Ile Ile Gly Ala His Phe
               180                 185                 190
   aac att gat aga aaa cca tca aat gag att cat gaa aat atc ata aaa     624
   Asn Ile Asp Arg Lys Pro Ser Asn Glu Ile His Glu Asn Ile Ile Lys
           195                 200                 205
   cat gaa aat tac agg gaa aga ata aaa agt gct gag aga aag gcg aaa     672
   His Glu Asn Tyr Arg Glu Arg Ile Lys Ser Ala Glu Arg Lys Ala Lys
       210                 215                 220
   aaa ctt gag gag cta tca agg aat gca aac ata aag ggt atc ttt gaa      720
   Lys Leu Glu Glu Leu Ser Arg Asn Ala Asn Ile Lys Gly Ile Phe Glu
   225                 230                 235                 240
   ctt gca gaa tcc gat aca gtg gaa tac cat aaa atg ctc cat gat gtt      768
   Leu Ala Glu Ser Asp Thr Val Glu Tyr His Lys Met Leu His Asp Val
                   245                 250                 255
   ggc gtt gac ata ata aat gat aga atg gag aac ctc att gaa agg gta      816
   Gly Val Asp Ile Ile Asn Asp Arg Met Glu Asn Leu Ile Glu Arg Val
               260                 265                 270
   aaa gaa atg aaa aat aac ttc tgg aat tca tac ata gtt acc ggc ggc      864
   Lys Glu Met Lys Asn Asn Phe Trp Asn Ser Tyr Ile Val Thr Gly Gly
           275                 280                 285
   ccg aac gtt ttt gta ata aca gag aaa aag gac gtt gat aag gca atg      912
   Pro Asn Val Phe Val Ile Thr Glu Lys Lys Asp Val Asp Lys Ala Met
       290                 295                 300
   gaa gga tta aat gat ctg tgc gat gat ata aga tta tta aaa gtt gca      960
   Glu Gly Leu Asn Asp Leu Cys Asp Asp Ile Arg Leu Leu Lys Val Ala
   305                 310                 315                 320
   gga aag cca cag gtc att tca aaa aac ttt taa                          993
   Gly Lys Pro Gln Val Ile Ser Lys Asn Phe
                   325                 330
<210> 20
   <211> 330
   <212> PRT
   <213> Artificial sequence
<220>
   <223> Synthetic Construct
<400> 20
   Met His His His His His His Glu Asn Tyr Asn Val Lys Thr Arg Ala
   1               5                   10                  15
   Phe Pro Thr Ile Gly Ile Ile Leu Leu Gly Gly Ile Ser Asp Lys Lys
               20                  25                  30
   Asn Arg Ile Pro Leu His Thr Thr Ala Gly Ile Ala Tyr Thr Gly Ile
           35                  40                  45
   Asn Asn Asp Val Tyr Thr Glu Thr Lys Leu Tyr Val Ser Lys Asp Glu
       50                  55                  60
   Lys Cys Tyr Ile Asp Gly Lys Glu Ile Asp Leu Asn Ser Asp Arg Ser
   65                  70                  75                  80
   Pro Ser Lys Val Ile Asp Lys Phe Lys His Glu Ile Leu Met Arg Val
                   85                  90                  95
   Asn Leu Asp Asp Glu Asn Asn Leu Ser Ile Asp Ser Arg Asn Phe Asn
               100                 105                 110
   Ile Leu Ser Gly Ser Ser Asp Ser Gly Ala Ala Ala Leu Gly Glu Cys
           115                 120                 125
   Ile Glu Ser Ile Phe Glu Tyr Asn Ile Asn Ile Phe Thr Phe Glu Asn
       130                 135                 140
   Asp Leu Gln Arg Ile Ser Glu Ser Val Gly Arg Ser Leu Tyr Gly Gly
   145                 150                 155                 160
   Leu Thr Val Asn Tyr Ala Asn Gly Arg Glu Ser Leu Thr Glu Pro Leu
                   165                 170                 175
   Leu Glu Pro Glu Ala Phe Asn Asn Phe Thr Ile Ile Gly Ala His Phe
               180                 185                 190
   Asn Ile Asp Arg Lys Pro Ser Asn Glu Ile His Glu Asn Ile Ile Lys
           195                 200                 205
   His Glu Asn Tyr Arg Glu Arg Ile Lys Ser Ala Glu Arg Lys Ala Lys
       210                 215                 220
   Lys Leu Glu Glu Leu Ser Arg Asn Ala Asn Ile Lys Gly Ile Phe Glu
   225                 230                 235                 240
   Leu Ala Glu Ser Asp Thr Val Glu Tyr His Lys Met Leu His Asp Val
                   245                 250                 255
   Gly Val Asp Ile Ile Asn Asp Arg Met Glu Asn Leu Ile Glu Arg Val
               260                 265                 270
   Lys Glu Met Lys Asn Asn Phe Trp Asn Ser Tyr Ile Val Thr Gly Gly
           275                 280                 285
   Pro Asn Val Phe Val Ile Thr Glu Lys Lys Asp Val Asp Lys Ala Met
       290                 295                 300
   Glu Gly Leu Asn Asp Leu Cys Asp Asp Ile Arg Leu Leu Lys Val Ala
   305                 310                 315                 320
   Gly Lys Pro Gln Val Ile Ser Lys Asn Phe
                   325                 330